(19) Europäisches Patentamt / European Patent Office / Office européen des brevets

(11) EP 0 595 133 B1

(12) EUROPÄISCHE PATENTSCHRIFT

(45) Veröffentlichungstag und Bekanntmachung des Hinweises auf die Patenterteilung:
**05.01.2011 Patentblatt 2011/01**

(51) Int Cl.:
***A61K 47/48*** (2006.01) ***C07H 13/08*** (2006.01)
***C07H 13/12*** (2006.01) ***C07H 15/203*** (2006.01)

(21) Anmeldenummer: **93116702.7**

(22) Anmeldetag: **15.10.1993**

(54) **Prodrugs, ihre Herstellung und Verwendung als Arzneimittel**

Prodrugs, their preparation and use as medicaments

Promédicaments, leur préparation, et utilisation comme médicaments

(84) Benannte Vertragsstaaten:
**AT BE CH DE DK ES FR GB GR IE IT LI LU NL PT SE**

(30) Priorität: **27.10.1992 DE 4236237**

(43) Veröffentlichungstag der Anmeldung:
**04.05.1994 Patentblatt 1994/18**

(73) Patentinhaber:
- **Sanofi-Aventis Deutschland GmbH**
  **65929 Frankfurt am Main (DE)**
- **Aventis Pharma S.A.**
  **92160 Antony (FR)**

(72) Erfinder:
- **Bosslet, Klaus Dr.**
  **D-35037 Marburg (DE)**
- **Czech, Jörg Dr.**
  **D-35041 Marburg (DE)**
- **Hoffmann, Dieter Dr.**
  **D-35041 Marburg (DE)**
- **Kolar, Cenek Dr.**
  **D-35037 Marburg (DE)**
- **Tillequin, Francois Dr.**
  **F-75013 Paris (FR)**
- **Florent, Jean-Claude Dr.**
  **F-91940 Les Ulis (FR)**
- **Azoulay, Michel Dr.**
  **F-75006 Paris (FR)**
- **Monneret, Claude Prof.**
  **F-75012 Paris (FR)**
- **Jacquesy, Jean-Claude Prof.**
  **F-86360 Buxerolles (FR)**
- **Gesson, Jean-Pierre Prof.**
  **F-86360 Chansseneuil du Poitou (FR)**
- **Koch, Michel Prof.**
  **F-78170 La Celle Saint Cloud (FR)**

(74) Vertreter: **Fischer, Hans-Jürgen et al**
**Sanofi-Aventis Deutschland GmbH**
**Patent- und Lizenzabteilung**
**Industriepark Höchst**
**Gebaüde K 801**
**65926 Frankfurt am Main (DE)**

(56) Entgegenhaltungen:
**EP-A- 0 410 366** **EP-A- 0 441 218**
**WO-A-81/01145** **WO-A-92/19639**
**GB-A- 1 541 436** **US-A- 4 975 278**

- **SUBR V: "POLYMERS CONTAINING ENZYMATICALLY DEGRADABLE BONDS XII. EFFECT OF SPACER STRUCTURE ON THE RATE OF RELEASE OF DAUNOMYCIN AND ADRIAMYCIN FROM POLY(N-(2-HYDROXYPROPYL)-METHACRYLAMIDE) COPOLYMER DRUG CARRIERS INVITRO AND ANTITUMOUR ACTIVITY MEASURED IN VIVO" JOURNAL OF CONTROLLED RELEASE, Bd. 18, Nr. 2, 1. Februar 1992, Seiten 123-132, XP000247009**
- **YUICHI OHYA ET AL: "SYNTHESIS AND CYTOTOXIC ACTIVITY OF DOXORUBICIN BOUND TO POLY(A-MALIC ACID) VIA ESTER OR AMIDE BONDS" MAKROMOLEKULARE CHEMIE, MACROMOLECULAR CHEMISTRY AND PHYSICS, Bd. 193, Nr. 8, 1. August 1992, Seiten 1881-1887, XP000371937**
- **DUNCAN R ET AL: "PRECLINICAL EVALUATION OF POLYMER-BOUND DOXORUBICIN" JOURNAL OF CONTROLLED RELEASE, Bd. 19, Nr. 1 / 03, 1. März 1992, Seiten 331-346, XP000261549**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die Erfindung betrifft Glykosyl-Spacer-Wirkstoff-Verbindungen (Prodrugs), ihre Herstellung sowie ihre Verwendung als Arzneimittel.

Die Therapie bösartiger Tumore, entzündlicher Erkrankungen, oder Autoimmunerkrankungen ist neben der unzureichenden Wirksamkeit der Therapeutika meist mit starken Nebenwirkungen verbunden. Dieser Mangel kann hauptsächlich mit der zu geringen in vivo Selektivität der eingesetzten Wirkstoffe erklärt werden. So lassen sich in vielen Fällen die günstigen in vitro pharmakologischen Eigenschaften der Wirkstoffe in vivo nicht bestätigen.

Seit vielen Jahren beschäftigen sich Wissenschaftler, allerdings ohne durchgreifenden Erfolg, mit diesem Problem. Eine Forschungsrichtung beschäftigte sich mit der Herstellung und Verwendung von Substanzen, die in vivo zu Prodrugs metabolisiert werden, die anschliessend ortsspezifisch durch Enzyme zu den Wirkstoffen (drugs) gespalten werden. So benutzten Sweeney und Mitarbeiter (Cancer Research 31, 477-478, 1971) Mycophenolsäure, die in vivo zu inertem Mycophenolsäureglucuronid metabolisiert wird, zur Behandlung von bösartigen Tiertumoren. Die beobachteten Effekte auf das Tumorwachstum erklären die Autoren durch enzymatische Abspaltung von Glucuronsäure durch im Tumor extrazellulär, d.h. auf der Tumorzellmembran vorhandene Glucuronidase, gefolgt von der Aufnahme von Mycophenolsäure in die Tumorzellen. Vom Konzept her identische Therapieversuche haben Young et al. (Cancer 38, 1887-1895, 1976) mit Anilinsenfgas im Rahmen einer klinischen Prüfung durchgeführt. Sie behandelten Tumorpatienten, deren Tumore auf hohe ß-Glucuronidasewerte geprüft wurden, mit Anilinsenfgas, welches entsprechend ihrer Hypothese in der Leber nach Hydroxilierung glucuronidiert und am Tumor durch ß-Glucuronidase zu toxischem Hydroxyanilinsenfgas gespalten werden sollte. Die therapeutischen Erfolge waren allerdings eher enttäuschend.

Eine weitere Forschungsrichtung ging einen Schritt weiter und stellte chemisch z.B. Anilinsenfgasglucuronsäuremethylester oder 6-Mercaptopuringlucuronid her. Diese Prodrugs zeigten allerdings nur einen niedrigen Detoxifizierungsgrad, was einer in vivo Anwendung entgegenstand. Günstigere Eigenschaften zeigten die 5-Fluorouracil O-β-D-glucuronid (FUOG)- oder 5-Fluorouracil N-glucuronid (FUNG) Verbindungen einer japanischen Arbeitsgruppe (Baba et al. Gann, 69, 283-284, 1978). Die Glucuronidierung von 5-Fluorouracil erhöhte die LD50 von 200 mg/kg für 5-FU auf 5000 mg/kg für das entsprechende Glucuronid. Deutliche Wirkung wurde allerdings nur mit FUOG nach zehnmaliger i.v. Gabe und Glucoseansäuerung bei der Behandlung eines Mausmammakarzinoms erhalten. Allerdings muß hier betont werden, daß die Behandlung bereits 24 Stunden nach Implantation eines Tumorstückchens begonnen wurde, ein Zeitpunkt, zu dem mit Sicherheit noch nicht von einem etablierten Tumor gesprochen werden kann. Wahrscheinlich bedingt durch potentielle Gewebezerstörung während der Implantation wurde im Tumor lysosomale Glucuronidase freigesetzt, die kombiniert mit der durch Glucosebehandlung verbundenen pH Absenkung zu in vivo Wirksamkeit führte. Die Relevanz dieses Modells für die klinische Situation erscheint allerdings sehr fraglich. Die Tatsache, daß bis heute noch kein Therapeutikum aus diesen Verbindungen entstanden ist, deutet auf die geringe in vivo Wirksamkeit der Verbindungen hin.

Eine Verbesserung der Wirksamkeit von Prodrugs hat sich die Arbeitsgruppe um Katzenellenbogen (WO 81/01145) dadurch versprochen, daß sie anstatt der Glucuronyl-drugs Peptidspacer-drugs synthetisiert hat. Als aktivierende Enzyme sollen hierbei tumorassoziierte fibrinolytische oder koagulierende Proteasen wie z.B. Plasmin oder Plasminogenaktivatoren dienen. In vivo pharmakologische Wirksamkeit ist weder mit den in WO 81/ 01145 noch mit den in Journal of Medicinal Chemistry, 24, 479-480 (1981) beschriebenen, durch Proteasen in vitro aktivierbaren Doxorubicin- und Arabinosylcytosinspacerpeptiden gezeigt worden. Vor dem Hintergrund unseres heutigen Wissens über das ubiquitäre extrazelluläre Vorkommen oben genannter Proteasen im menschlichen Körper ist die fehlende selektive in vivo Wirksamkeit der oben synthetisierten Verbindungen erklärbar.

Trotz einer Vielzahl von Hinweisen aus der oben zitierten Literatur über die nicht sehr erfolgreiche Verwendung von Glucuronsäure-haltigen Prodrugs, auch in Kombination mit Glucoseansäuerung (Baba, T. et al., Gann 69, 283-284, 1978) erhielt Rubin im Jahre 1984 ein US Patent (No. 4 481 195), in welchem er die Verwendung von Glucuronsäure-Wirkstoffverbindungen nach Ansäuerung des Tumors und Alkalisierung des Normalgewebes vorschlägt. Aus dieser Erfindung sind anscheinend noch keine verwendbaren klinisch erfolgreichen Therapeutika hervorgegangen.

Außerdem wurden durch Esterasen spaltbare Buttersäure-Prodrugs beschrieben. Allerdings zeigte sich, daß die therapeutischen Effekte der aus den Prodrugs in vivo freigesetzten Buttersäure denen eines Standardzytostatikums (Cisplatin) unterlegen sind.

Alle soweit diskutierten Arbeiten gehen von einer Aktivierung von Prodrugs durch körpereigene Enzyme aus. Die mit diesem Prinzip erzielbaren in vivo-therapeutischen Effekte scheinen aber der Standard-Chemotherapie nicht überlegen zu sein.

Unabhängig von der bisher beschriebenen Forschungsrichtung (Prodrug-Aktivierung durch körpereigene Enzyme) entwickelte sich eine neue Forschungsrichtung, die versuchte nach Prälokalisation von xenogenen Antikörperenzymkonjugaten im Zielgewebe Prodrugs selektiv im Zielgewebe zu zytotoxischen Drugs zu spalten. (Philpott et al. Surgery 74, 51, 1973; Cancer Res. 34, 2159, 1974). Bagshawe (WO 88/07378) schlug, basierend auf den Arbeiten von Philpott, die Verwendung von xenogenen Antikörperenzymkonjugaten in Kombination mit Prodrugs zur Behandlung von

Tumoren vor. Er verwendete Mausmonoklonale Antikörper, die er chemisch an bakterielle Carboxypeptidase G2 koppelte, als Antikörperenzymkonjugat und Glutamylsenfgas als Prodrug. Senter (USP 4,975,278) beschreibt die Kombination von Antikörperenzymkonjugaten bestehend aus Maus-monoklonalen Antikörpern chemisch gebunden an alkalischer Phosphatase mit Etoposidphosphat bzw. Penicillin V-amidase und N(-p-hydroxyphenoxyacetyl)adriamycin Prodrugs. Beide Systeme (Bagshawe und Senter) haben den Nachteil, daß die verwendeten Antikörperenzymkonjugate xenogen und damit hoch immunogen sind. Dadurch ist wahrscheinlich nicht die Möglichkeit gegeben, sie wiederholt am gleichen Patienten im Rahmen mehrerer Therapiezyklen einzusetzen. Zusätzlich besteht im System von Senter der Nachteil, daß das Enzym schon in erheblicher Menge im Humanblut vorkommt, wodurch eine systemische Aktivierung der Prodrug erfolgt.

Aus diesen Unzulänglichkeiten heraus wurde von Bosslet et al. (Br. J. Cancer 65, 234-238, 1992) ein Fusionsprotein, bestehend aus dem humanisierten F(ab') Fragment eines anti CEA Antikörpers und der humanen ß-Glucuronidase, hergestellt, welches enzymatisch aktiv ist, Tumorselektivität besitzt, Glucuronyl-drugs spalten kann (Florent et al., Int. Carbohydr. Symposium p. 297, Abstract A262, Paris, 1992; Gesson et al., Int. Carbohydr. Symposium, p. 298, Abstract A263, Paris, 1992; Andrianomenjanahary et al., Int. Carbohydr. Symposium, p. 299, Abstract A264, Paris, 1992) und nach heutigem Stand des Wissens nicht oder nur sehr wenig immunogen ist.

EP 0 441 218 A2 beschreibt Anthracyclin-Glykosyl-Prodrugs, worin die Glykosyleinheit direkt mit dem Anthracyclin verbunden ist, und ihre Anwendung in Kombination mit funktionalisierten tumorspezifischen Enzymkonjugaten für die Behandlung von Krebserkrankungen. Die Prodrugs werden dabei durch die Enzymkonjugate zu zytotoxischen Wirkstoffen gespalten.

Im Rahmen der Arbeiten zur Synthese und in vivo pharmakologischen Prüfung von Verbindungen, die optimal von diesem Fusionsprotein gespalten werden sollten, haben wir unerwarteter Weise Substanzen gefunden, die in Tumoren mit deutlichem Anteil desintegrierender Zellen, in entzündlichen Prozessen und bei Autoimmunerkrankungen ohne vorherige Applikation des Fusionsproteins sehr effizient gespalten werden.

Hierbei werden zur Aktivierung der Verbindungen beim gesunden Individuum vornehmlich intrazellulär vorkommende Enzyme genutzt, die unter oben erwähnten pathophysiologischen Bedingungen lokal extrazellulär vorkommen.

Diese Verbindungen besitzen die allgemeine Formel Glykosyl-Spacer-Wirkstoff, dadurch gekennzeichnet, daß Glykosylrest und Spacer unter physiologischen oder pathophysiologischen Bedingungen vom Wirkstoff abspaltbar sind. Die Eigenschaften dieser Verbindungen sind derart, daß im allgemeinen der Poly, Oligo- oder Monoglykosylrest durch enzymatische Hydrolyse, und danach der Spacer durch chemische Hydrolyse spontan abgespalten wird. Wirkstoff bedeutet eine chemische Substanz mit biologischer Wirkung, besonders ein Pharmazeutikum, sowie deren durch Einführung zusätzlicher Hydroxy-, Amino- oder Iminogruppen gewonnenen Derivate.

Gegenstand der Erfindung sind solche Verbindungen und deren Verwendung.

Gegenstand der Erfindung sind besonders Glykosyl-Spacer-Wirkstoff-Verbindungen der Formel 1

$$\text{Glykosyl-Y[-C(= Y)-X-}J_p\text{-W(R)}_n\text{-X-C(=Y)-Wirkstoff} \qquad \text{(I)}$$

worin

| | |
|---|---|
| Glykosyl | ein enzymatisch abspaltbares Poly-,Oligo- oder Monosaccharid, |
| W | ein Aromat oder Heteroaromat oder Aliphat mit konjugierten Doppelbindungen oder eine nach Abspaltung des Glykosylrests zyklisierender Aminosäurederivatrest, mit vorzugsweise 5-20 Kohlenstoffatomen und 0-4 Heteroatomen, wobei Heteroatom N, O oder S bedeutet, ist, an den die Substituenten R gebunden sein können, wobei |
| R | unabhängig oder gleich H, Methyl, Methoxy, Carboxy, Methyloxycarbonyl, CN, Hydroxy, Nitro, Fluor, Chlor, Brom, Sulfonyl, Sulfonamid oder Sulfon $(C_{1-4})$-alkylamid sind und |
| p | 0 oder 1 |
| n | eine ganze Zahl, |
| X | O, NH, methylenoxy, methylenamino oder methylen $(C_{1-4})$-alkylamin |
| Y | O oder NH ist, und |
| Wirkstoff | eine über eine Hydroxy-, Amino- oder Iminogruppe verknüpfte Verbindung mit biologischer Wirkung, bevorzugt ein Pharmazeutikum, besonders bevorzugt ein über eine Hydroxy- oder bei p = 0 nicht 3'-Aminogruppe verknüpftes Anthrazyklin, vorzugsweise Doxorubicin, 4'-epi-Doxorubicin, 4- oder 4'-Desoxy-doxorubicin oder eine Verbindung vorzugsweise ausgewählt aus der Gruppe Etoposide, N-Bis-(2-chlorethyl)-4-hydroxyanilin, 4-Hydroxycyclophosphamid, Vindesin, Vinblastin, Vincristin, Terfenadin, Terbutalin, Fenoterol, Salbutamol, Muscarin, Oxyphenbutazon, Salicylsäure, p-Aminosalicylsäure, 5-Fluorouracil, 5-Fluorocytidin, 5- Fluorouridin, Methotrexat, Diclofenac, Flufenaminsäure, 4-Methylaminophenazon, Theophyllin, Nifedipin, Mitomycin C, Mitoxantron, Camptothecin, m-AMSA, Taxol, Nocodaxol, Colchicin, Cyclophospha- |

mid, Rachelmycin, Cisplatin, Melphalan, Bleomycin, Stickstoff-Senfgas, Phosphoramid-Senfgas, Quercetin, Genistein, Erbstatin, Tyrphostin, Rohitukin-Derivat ((-)-cis-5,7-Dihydroxy-2-(2-chlorphenyl)-8-[4-(3-hydroxy-1-methyl)-piperidinyl]-4H-1-benzopyran-4-on; EP 89119710.5), Retinolsäure, Buttersäure, Phorbolester, DMSO, Aclacinomycin, Progesteron, Buserelin, Tamoxifen, Mifepriston, Onapriston, N-(4-aminobutyl)-5-chloro-2-naphtalen-sulfonamid, Pyridinyloxazol-2-on, Quinolyl-, Isoquinolyloxazolone-2-on, Staurosporin, Ethanolamin, Verapamil, Forskolin, 1,9-Dideoxyforskolin, Quinin, Quinidin, Reserpin, 18-O-(3,5-dimethoxy-4-hydroxybenzoyl)-reserpat, Lonidamin, Buthioninsulfoximin, Diethyldithiocarbamat, Cyclosporin A, Azathioprin, Chlorambucil, N-(4-Trifluormethyl)-phenyl-2-cyano-3-hydroxy-croton-säureamid (WO 91/17748), 15-Deoxyspergualin, FK 506, Ibuprofen, Indomethacin, Aspirin, Sulfasalazin, Penicillinamin, Chloroquin, Dexamethason, Prednisolon, Lidocain, Propafenon, Procain, Mefonaminsäure, Paracetamol, 4-Aminophenazon, Muskosin, Orciprenalin, Isoprenalin, Amilorid, p-Nitrophenylguanidinobenzoat oder deren durch eine oder mehrere Hydroxy-, Amino-oder Iminogruppen zusätzlich substituierte Derivate bedeutet.

Bevorzugt sind Verbindungen der Formel I,
worin

W ein Phenylrest oder ein mehrfach substituierter Phenylrest, bei dem die Substituenten
R unabhängig oder gleich H, Methyl, Methoxy, Carboxy, Methyloxycarbonyl, CN, Hydroxy, Nitro, Fluor, Chlor, Brom, Sulfonyl, Sulfonamid oder Sulfon $(C_{1-4})$- alkylamid sind und
p 0 oder 1,
n 1 bis 4 ist,
X O, NH, methylenoxy, methylenamino oder methylen-$(C_{1-4})$-alkylamino,
Y O oder NH ist,

und
Wirkstoff eine Verbindung wie oben beschrieben bedeutet.
Besonders bevorzugte Verbindungen sind solche Verbindungen der Formel I, worin

Glykosyl ein Poly-,Oligo- oder Monosaccharid , insbesondere ein alpha- oder beta-O-glycosidisch verknüpfter D-Glucuronyl-, D-Glucopyranosyl-, D-Galactopyranosyl-, N-Acetyl-D-glucosaminyl-, N-Acetyl-D-galactosaminyl-, D-Mannopyranosyl- oder L-Fucopyranosylrest,
W ein Phenylrest oder ein monosubstituierter Phenylrest, bei dem einer der Substituenten
R Methoxy, Methyloxycarbonyl, CN, Hydroxy, Nitro, Fluor, Chlor, Brom, Sulfonyl oder Sulfonamid ist und die übrigen Wasserstoff sind,
X O, NH, methylenoxy, methylenamino oder methylen-methylamino und
Y O oder NH ist und

Wirkstoff eine Verbindung wie oben beschrieben bedeutet.
Bevorzugte Ausführungsformen der Erfindung sind die folgenden:

Verbindung, dadurch gekennzeichnet, daß der Glykosylrest durch enzymatische Hydrolyse abgespalten werden kann, daß der Spacer durch chemische Hydrolyse spontan abgespalten werden kann, daß der Wirkstoff ein Pharmazeutikum oder eines seiner durch Einführung zusätzlicher Hydroxy-, Amino- oder Iminogruppen erhaltenen Derivate ist, daß sie hydrophiler ist als der Wirkstoff, daß sie in vivo zu weniger toxischen Reaktionen führt als der Wirkstoff als solcher, daß der Wirkstoff eine pharmakologisch wirksame Substanz ist, daß der Wirkstoff durch eine oder mehrere Hydroxy-, Amino- oder Iminogruppe zusätzlich substituiert ist und das Tumorwachstum verlangsamt, daß der Wirkstoff ein Standardzytostatikum ist, daß der Wirkstoff ein Antimetabolit ist, daß der Wirkstoff 5-Fluorouracil, 5-Fluorocytidin, 5-Fluorouridin, Cytosinarabinosid oder Methotrexat ist, daß der Wirkstoff eine in die DNA interkalierende Substanz ist, daß der Wirkstoff Doxorubicin, Daunomycin, Idarubicin, Epirubicin oder Mitoxantron ist, daß der Wirkstoff die Topoisomerase I + II hemmt, daß der Wirkstoff Camptothecin, Etoposid oder M-AMSA ist, daß der Wirkstoff ein Tubulinhemmer ist, daß der Wirkstoff Vincristin, Vinblastin, Vindesin, Taxol, Nocodaxol, Colchicin oder Etoposid ist, daß der Wirkstoff ein Alkylanz ist, daß der Wirkstoff Cyclophosphamid, Mitomycin C, Rachelmycin, Cisplatin, Phosphoramid-Senfgas, Melphalan, Bleomycin, Stickstoff-Senfgas oder N-Bis-(2-chlorethyl-4-hydroxyanilin ist, daß der Wirkstoff Neocarzinostatin, Calicheamicin, Dynemicin oder Esperamicin A ist, daß der Wirkstoff eine die Ribosomen inaktivierende Verbindung ist, daß der Wirkstoff Verrucarin A ist, daß der Wirkstoff ein Tyrosinphosphokinaseinhibitor ist, daß der Wirkstoff Quercetin, Genistein, Erbstatin, Tyrphostin oder Rohitukin-Derivat ist, daß der Wirkstoff ein Differenzierungsinduktor ist, daß der Wirkstoff Retinolsäure, Buttersäure, Phorbolester, DMSO oder Aclacinomycin ist, daß der Wirkstoff ein Hormon, Hormonagonist bzw. Hormonantagonist ist,

daß der Wirkstoff Progesteron, Buserelin, Tamoxifen, Mifepriston oder Onapriston ist, daß der Wirkstoff eine Substanz ist, welche die pleiotrope Resistenz gegenüber Zytostatika verändert, daß der Wirkstoff ein Calmodulin-Inhibitor ist, daß der Wirkstoff ein Proteinkinase C-Inhibitor ist, daß Wirkstoff ein P-Glykoprotein-Inhibitor ist, daß der Wirkstoff ein Modulator der mitochondrial gebundenen Hexokinase ist, daß der Wirkstoff ein Inhibitor der γ-Glutamylcysteinsynthetase oder der Glutathion-S-transferase ist, daß der Wirkstoff ein Inhibitor der Superoxiddismutase ist, daß der Wirkstoff einen Inhibitor des durch den MAk Ki67 definierten proliferationsassoziierten Proteins im Zellkern sich teilender Zellen darstellt, daß der Wirkstoff eine Substanz ist, die immunsuppressive Effekte ausübt, daß der Wirkstoff ein Standardimmunsuppressivum ist, daß der Wirkstoff ein Makrolid ist, daß der Wirkstoff Cyclosporin A, Rapamycin, FK 506 ist, daß der Wirkstoff Azathioprin, Methotrexat, Cyclophosphamid oder Chlorambucil ist, daß der Wirkstoff eine Substanz ist, die antiinflammatorische Wirkung hat, daß der Wirkstoff eine nicht steroidale antiinflammatorische Substanz ist, daß der Wirkstoff eine slow acting anti-rheumatic drug ist, daß der Wirkstoff ein Steroid darstellt, daß der Wirkstoff eine Substanz ist, die antiphlogistische, analgetische oder antipyretische Wirkung hat, daß der Wirkstoff ein Derivat einer organischen Säure darstellt, daß der Wirkstoff ein nicht saures Analgetikum / Antiphlogistikum darstellt, daß der Wirkstoff Oxyphenbutazon ist, daß der Wirkstoff ein Lokalanästhetikum ist, daß der Wirkstoff ein Antiarrhythmikum ist, daß der Wirkstoff ein $Ca^{++}$ Antagonist ist, daß der Wirkstoff ein Antihistaminikum ist, daß der Wirkstoff ein Hemmstoff der Phosphodiesterase ist, daß der Wirkstoff ein Parasympathomimetikum ist, daß der Wirkstoff ein Sympathomimetikum ist oder daß der Wirkstoff eine Substanz mit inhibitorischer Wirkung auf die humane Urokinase ist; und außerdem Verbindung, dadurch gekennzeichnet, daß der

Glykosylrest ein alpha- oder beta-O-glycosidisch verknüpfter D-Glucuronyl-, D-Glucopyranosyl-, D-Galactopyranosyl-, N-Acetyl-D-glucosaminyl-, N-Acetyl-D-galactosarninyl-, D-Mannopyranosyl- oder L-Fucopyranosylrest ist, daß sie 4'-O-[4-(alpha-D-Glucopyranosyloxy)-phenylaminocarbonyl]-etoposid, 4'-O-[4-(beta-D-Glucopyranosyloxy)-phenylaminocarbonyl]-etoposid, 4'-O-[4-(aipha-D-Galactopyranosyloxy)-phenylaminocarbonyl]- etoposid, 4'-O-[4-(beta-D-Glucuronyloxy)-phenylaminocarbonyl]-etoposid, 4'-O-[4-(beta-D-Glucuronyloxy)-3-nitro-benzylaminocarbonyl]- etoposid, 4'-O-[4-(beta-D-Glucuronyloxy)-3-chlor-benzylaminocarbonyl]- etoposid, 1-N-[4-(beta-D-Glucuronyloxy)-benzyloxycarbonyl]-mitomycin C, 14-O-[4-(beta-D-Glucuronyloxy)-3-nitro-benrylaminocarbonyl]-doxorubicin, 4-O-[4-(beta-D-Glucuronyloxy)-benzylaminocarbonyl]-4-hydroxy-1-N-(bis-2-chlorethyl)-anilin, 4-O-[4-(beta-D-Glucuronyloxy)-benzylaminocarbonyl]-terfenadin, 3'-O-[4-(beta-D-Glucuronyloxy)-benzylaminocarbonyl]-terbutalin, 3'-O-[4-(beta-D-Glucuronyloxy)-benzylaminocarbonyl]-fenoterol, 1"-O-[4-(beta-D-Glucuronyloxy)-benzylaminocarbonyl]-salbutamol, 3-O-[4-(beta-D-Glucuronyloxy)-benzylaminocarbonyl]-muscarin, 4'-O-[4-(beta-D-Glucuronyloxy)-benzylaminocarbonyl]-oxyphenbutazon, 2-O-[4-(beta-D-Glucuronyloxy)-benzylaminocarbonyl]-salicylsäure, N-[4-(beta-D-Glucuronyloxy)-benzyloxycarbonyl]-diclofenac, N-[4-(beta-D-Glucuronyloxy)-benzyloxycarbonyl]- flufenaminsäure, 4-N-[4-(beta-D-Glucuronyloxy)-benzyloxycarbonyl]-4-methyl-aminophenazon, 7-N-[4-(beta-D-Glucuronyloxy)-benryloxycarbonyl]-theophyllin, 1-N-[4-(beta-D-Glucuronyloxy)-benzyloxycarbonyl]-nifedipin, [4-(β-D-glucuronyloxy)-3-nitrobenzyl]-2-[1-cyano-1-(N-4-trifluormethylphenyl)-carbamoyl]propen-1-yl-carbonat, 3'-N-[4-N-(alpha-D-Galactosyloxycarbonyl)-4-aminobenzyloxycarbonyl]-doxorubicin, 9-O-[4-(beta-D-Glucuronyloxy)-3-chlorobenzyloxycarbonyl]-quinin oder 18-O-[3,5-Dimethoxy-4-[4-(beta-D-glucuronyloxy) -3-chlorobenzyloxycarbonyl] benzoyl]-reserpat ist.

Diese Verbindungen können in eine geeignete galenische Darreichungsform (z.B. Liposomen oder geträgert auf humane Proteine) gebracht werden und als Arzneimittel gebraucht werden.

Die pharmakologische Wirksamkeit von erfindungsgemäßen Glykosyl-Spacer-Wirkstoff-Verbindungen (im folgenden Prodrug genannt) wurde in vivo in relevanten tierexperimentellen Systemen getestet. Für die onkologische Indikation wurde ein Modell ausgewählt, in dem humane Tumore subkutan auf nackte Mäuse transplantiert werden und die erfindungsgemäßen Prodrugs nach Etablierung des Tumors i. v. appliziert werden.

Die Ergebnisse (Beispiele 26 - 28 mit Tabellen) zeigen, daß die erfindungsgemäßen Prodrugs bei Tumoren mit signifikantem desintegrierendem Tumorzellanteil wesentlich wirksamer sind als die Standardchemotherapie, durchgeführt mit der maximal verträglichen Dosis an Drug. Hierbei ist es unbedeutend, ob die Desintegration eines signifikanten Tumorzellanteils durch die Tumorgröße und die daraus resultierende mangelhafte Ernährung von Teilen des Tumors (zentrale Nekrose) bewirkt wird oder durch eine exogen zugeführte Substanz (Immuntoxin, Bestrahlung, Fusionsprotein als überlegene Ausführungsform eines Antikörperenzymkonjugates, Fusionsprotein aus einer Binderegion und der DNAse 1, z.B. scFVDNAseI, Zytostatikum, etc.) in einem vorhergehenden, parallel ablaufenden, oder danach eingesetzten Behandlungsschritt induziert wird. Die überlegene pharmakologische Wirksamkeit der erfindungsgemäßen Prodrugs ist durch folgende Eigenschaften bedingt:

a) Die Prodrugs sind in vivo signifikant (> 70x) weniger toxisch als die in der Prodrug enthaltenen Standarddrugs.
b) Die in vivo am Ort der Aktivierung (Tumor) aus der Prodrug freigesetzte zytotoxische Drugmenge ist unter obigen experimentellen Bedingungen 5-50x höher als die durch i.v. Standardtherapie im Tumor erreichbare Drugmenge.

c) Die in Normalgeweben unspezifisch aus der Prodrug freigesetzte Drugmenge bzw. durch potentielle Ausschwemmung von der am Tumor erzeugten Drug bedingte Drugkonzentration in den Normalgeweben liegt deutlich unter den Konzentrationen der Normalgewebedrug, die durch Applikation von Standarddrugs nach i.v. Gabe erreicht werden. Diese Beobachtung untermauert die Daten, welche die drastisch reduzierte in vivo Toxizität der Prodrug gegenüber der Drug belegen (a).

d) Plasmapharmakokinetische Untersuchungen sowie Urinanalysen zeigen, daß die erfindungsgemäßen Prodrugs in gesunden Tieren sehr schnell (t1/2 ≈ 12 min) hauptsächlich über die Nieren als ungespaltene Prodrugs ausgeschieden werden.

Diese Beobachtungen lassen den Schluß zu, daß die erfindungsgemäßen Prodrugs eine ausreichende Hydrophilie besitzen, die eine hauptsächlich extrazelluläre Verteilung in vivo bedingt.

Da der Glykosylanteil der erfindungsgemäßen Prodrugs so gewählt ist, daß er nur von unter pathophysiologischen Bedingungen lokal freigesetzten Enzymen abspaltbar ist, kann die lipophile Drug ebenfalls nur am Zielgewebe freigesetzt werden und dort ihre zytotoxische Wirkung entfalten.

Steigern läßt sich die überlegene Wirkung einer erfindungsgemäßen Prodrug mit zytotoxischer Drugkomponente dadurch, daß sie mit erfindungsgemäßen Prodrugs mit einer anderen zytotoxischen Drugkomponente kombiniert wird. Hierbei sind Prodrugkombinationen von Vorteil, bei denen zytotoxische Komponenten mit unterschiedlichem Wirksamkeitsmechanismus verwendet werden, entsprechend der Poly-Chemotherapie. Besonders geeignet erscheint der Einsatz von Wirkstoffen, die sehr effizient Einzelstrangund Doppelstrangbrüche in der DNA hervorrufen wie Calicheamicin. Von besonderem Vorteil sind allerdings erfindungsgemäße Prodrugkombinationen, bei denen eine Drug zytotoxisches Potential hat, eine andere aber z.B. die Multi-Drug-Resistenz blockiert.

Besonders geeignet sind in diesem Zusammenhang erfindungsgemäße Prodrugs, deren Drugkomponente die Multi-Drug-Resistenz dadurch beeinflußt, daß sie die Tyrosinphosphokinase inhibiert, die Differenzierung induziert, Hormon- bzw. Hormonantagonistische Wirkung zeigt, ein Calmodulin-Inhibitor ist, ein Proteinkinase C-Inhibitor ist, ein P-Glykoproteininhibitor ist, ein Ionenkanalblocker ist, die mitochondriale Hexokinase inhibiert, die gamma-Glutamylcysteinsynthetase, die Glutathion-S-transferase sowie die Superoxiddismutase inhibiert. Weitere interessante Wirkstoffe zur Beeinflussung des Tumorwachstums sind Verbindungen, die das von Gerdes et al. (Amer. J. Pathol. 138, 867-873, 1991) beschriebene proliferationsinduzierte Protein funktionell blockieren. Besonders als Wirkstoffkomponente in den erfindungsgemäßen Glykosyl-Spacer-Wirkstoff-Verbindungen sollte sich die Effizienz dieser Wirkstoffe nach lokaler enzymatischer Aktivierung besonders selektiv nutzen lassen.

Besonders günstige therapeutische Effekte werden erzielt, wenn z.B. eine Glucuronyl-Spacer-Quinin Prodrug kombiniert mit einer Glucuronyl-Spacer-Doxorubicin Prodrug (siehe Tabelle 1) eingesetzt wird. Analytische Untersuchungen ergaben, daß in einer solchen Kombination die Quininkonzentration im Tumor ähnlich stark erhöht ist im Vergleich zu konventioneller Quininbehandlung wie die zytostatische Drugkonzentration im Vergleich zu konventioneller Zytostatikatherapie.

Für die pharmakologische Prüfung erfindungsgemäBer Prodrugs, die für nicht onkologische Erkrankungen geeignet sind, wurden folgende tierexperimentelle Systeme ausgewählt:

a) Granuloma pouch Modell in der Maus:

Bottomley et al., Brit. J. Pharmacol. 93, 627-635 (1988)

b) Adjuvansarthritis in der Ratte Schorlemmer et al., Exptl. Clin. Res. XVII (10/11) 471-483 (1991)

c) DTH-Modell in der Maus Dickneite et al., Infect. Immun. 44 (1), 168-174 (1984)

d) Dextransulfat-induzierte Colitis in der Maus Okayasu et al., Gastroenterology 98, 694-702 (1990)

e) EAE-Modell Schorlemmer et al., Drugs Exptl. Clin. Res. XVII (10/11) 461-469 (1991)

f) MRL-1 Modell Schorlemmer et al., Int. J. Immunother. VII (4) 169-180 (1991)

In diesen Modellen wurde die Wirksamkeit, vor allem der in Beispiel 8 und 22 näher beschriebenen Prodrug-Verbindung 8 bzw. 2 2untersucht. Überlegene Wirksamkeit der Prodrug 22 gegenüber dem Wirkstoff (Leflunomid ) selbst wurde vor allem bei der Adjuvansarthritis und der EAE festgestellt. Überlegene Wirkung von Prodrug 8 gegenüber Standardtherapie wurde bei der DTH festgestellt. Ähnlich wie in den vorhergehenden Untersuchungen in der onkologischen Indikation war die überlegene Wirksamkeit mit höheren Wirkstoffkonzentrationen vor allem in der Synovialflüssigkeit verbunden (Adjuvansarthritis).

Diese Beobachtungen in den oben erwähnten nicht onkologischen Modellen legen die generelle Verwendbarkeit der erfindungsgemäßen Prodrugs nahe. Als Drugkomponente kommen alle Substanzen in Frage, deren therapeutischer Einsatz mit unangenehmen Nebenwirkungen verbunden ist, bzw. deren Wirkkonzentration in vivo nur grenzwertig erreicht wird. Hierzu zählen neben dem bereits in Prodrugform verwendeten immunsuppressiven Wirkstoff aus Beispiel 2[2] weitere

Immunsuppressiva (Azathioprin, Methotrexat, Cyclophosphamid, Chlorambucil,15 - Deoxy sperqualin, Cyclosporin A, FK 506 etc.), nicht steroidale antiinflammatorische Drugs (NSAIDs; Bsp.: Ibuprofen, Indomethacin, Aspirin etc.), slow acting anti-rheumatic drugs (SAADRs: Bsp.: Sulfasalazin, Penicillinamin, Chloroquin) und Steroide (Bsp.: Dexamethason, Prednisolon, etc.). Des weiteren eignen sich die im folgenden beispielhaft erwähnten Substanzen mit antiphlogistischer, analgetischer und antipyretischer Wirkung als Drugbaustein in den erfindungsgemäßen Prodrugs.

Beispiele für Substanzen mit antiphlogistischer, analgetischer und antipyretischer Wirkung:

1. Derivate organischer Säuren:

- Salicylsäure
- p. Aminosalicylsäure
- Diclofenac
- Flufenaminsäure
- Mefenaminsäure

2. Nicht saure Analgetika/Antiphlogistika

- Paracetamol
- Pyrazolon-Derivate (z.B. 4-Aminophenazon; 4-Methylaminophenazon)

3.

- Oxyphenbutazon

Weitere Drugs, die sich als Baustein für die erfindungsgemäßen Prodrugs eignen, sind die $Ca^{++}$ -Antagonisten (Bsp.: Nifedipin, Indikation: entzündliche Erkrankungen), die Antihistaminika (Bsp.: Terfenadin, Indikation: Allergie, Asthma, entzündliche Erkrankungen), Hemmstoffe der Phosphodiesterase (Bsp.: Theophyllin, Indikation: Asthma, Allergie, entzündliche Erkrankungen), Parasympathomimetika (Bsp.: Muskarin, Indikation: Autoimmunerkrankungen) sowie Sympathomimetika (Bsp.: Terbutalin, Fenoterol, Sulbutamol, Orciprenalin, Isoprenalin, Indikation: Asthma).
Eine als Wirkstoff für die erfindungsgemäßen Prodrugs besonders geeignete Substanzklasse stellen die synthetischen Urokinaseinhibitoren dar (wie z.B. p-Ntrophenylguanidinobenzoat, Amilorid etc.), die vorzugsweise bei der Behandlung entzündlicher Erkrankungen in Prodrugform zukünftig Verwendung finden könnten.

Zusammenfassend soll hier nochmals betont werden, daß die oben erwähnten Drugs bzw. die hieraus erfindungsgemäß herstellbaren Prodrugs nur Beispiele darstellen. Die erfindungsgemäßen Prodrugs lassen sich bei allen nicht onkologischen Erkrankungen einsetzen, bei denen Makrophagen, Granulozyten und Thrombozyten, insbesondere in aktiviertem Zustand vorkommen. In aktiviertem Zustand scheiden die oben erwähnten Zellen vornehmlich intrazelluläre Enzyme aus, die eine ortsspezifische Aktivierung der erfindungsgemäßen Prodrugs ermöglicht.

In der onkologischen Indikation erfolgt die Aktivierung der erfindungsgemäßen Prodrugs durch aus sterbenden Tumorzellen freigesetzte intrazelluläre Enzyme. Dieses Phänomen tritt vor allem bei größeren Tumoren auf (> 0.3 cm), aber auch nach Schädigung des Tumors durch Behandlung mit Immuntoxinen, Zytostatika, Bestrahlung, Fusionsproteinen, Antikörperenzymkonjugaten etc. Des weiteren kann ein Beitrag zur Prodrugaktivierung von im Tumor vorhandenen aktivierten Zellen (vor allem von Makrophagen, Granulozyten etc.) nicht ausgeschlossen werden.

Die folgenden Beispiele erläutern die Erfindung:

Beipiel 1

4'-O-[4-(alpha-D-Glucopyranosyloxy)-phenylaminocarbonyl]-etoposid (Verbindung 1)

500 mg (0.68 mmol) 2",3"-Di-O-chloracetyl-etoposid wurde in 50 ml DMF gelöst und mit 0.34 ml (3 äq) Diisopropylethylamin und 0.12 ml (1.5 äq) Diphosgen bei Raumtemperatur versetzt. Die Reaktionsmischung wurde 2 h gerührt und anschließend mit 0,22 ml Diisopropylethylamin und 250 mg (0.9 mmol) 4-(alpha-D-Glucopyranosyloxy)-anilin gelöst in 50 ml DMF versetzt. Der Reaktionsansatz wurde 14 h bei Raumtemperatur gerührt, dann mit Ethylacetat versetzt und dreimal mit Citratpuffer (pH 5) ausgewaschen. Die organische Phase wurde über Natriumsulfat getrocknet und in vacuo eingedampft. Der Rückstand wurde säulenchromatographisch über Kieselgel (50 g) mit Chloroform und Methanol 6:1 gereinigt. Es wurden 438 mg 4'-O-[4-(alpha-D-Glucopyranosyloxy)-phenylaminocarbonyl]-2",3"-di-O-chloracetyl-etoposid erhalten. Dünnschichtchromatographische Analyse: Rf= 0.6 in Chloroform und Methanol 3:1.
Das erhaltene Konjugat (400 mg) wurde in 80 ml Methanol gelöst und mit 3.0 g Ionenaustauscher Dowex 1x8 versetzt.

Die Reaktionsmischung wurde 4 h bei Raumtemperatur gerührt und das Harz wurde abfiltriert und nachgewaschen. Das Filtrat wurde in vacuo eingedampft. Der Rückstand wurde über Kieselgel (50 g) mit Chloroform, Methanol und Eisessig 5:2:2 gereinigt. Es wurden 256 mg Titelverbindung erhalten. Dünnschichtchromatographische Analyse: Rf= 0.46 in Chloroform, Methanol und Eisessig 5:2:2.

Beispiel 2

4'-O-[4-(beta-D-Glucopyranosyloxy)-phenylaminocarbonyl]-etoposid (Verbindung 2)

500 mg (0.68 mmol) 2",3"-Di-O-chloracetyl-etoposid wurde in 50 ml DMF gelöst und mit 0.34 ml (3 äq) Diisopropylethylamin und 0.12 ml (1.5 äq) Diphosgen bei Raumtemperatur versetzt. Die Reaktionsmischung wurde 2 h gerührt und anschließend mit 0,22 ml Diisopropylethylamin und 250 mg (0.9 mmol) 4-(beta-D-Glucopyranosyloxy)-anilin gelöst in 50 ml DMF versetzt. Der Reaktionsansatz wurde 14 h bei Raumtemperatur gerührt, dann mit Ethylacetat versetzt und dreimal mit Citratpuffer (pH 5) ausgewaschen. Die organische Phase wurde über Natriumsulfat getrocknet und in vacuo eingedampft. Der Rückstand wurde säulenchromatographisch über Kieselgel (50 g) mit Chloroform und Methanol 6:1 gereinigt. Es wurden 450 mg 4'-O-[4-(beta-D-Glucopyranosyloxy)-phenylaminocarbonyl]-2", 3"-di-O-chloracetyl-etoposid erhalten. Dünnschichtchromatographische Analyse: Rf = 0.56 in Chloroform und Methanol 3:1.
Das erhaltene Konjugat (400 mg) wurde in 80 ml Methanol mit 3.0 g Ionenaustauscher Dowex 1x8 entblockiert, wie im Beispiel 1 beschrieben. Es wurden 270 mg Titelverbindung erhalten. Dünnschichtchromatographische Analyse: Rf = 0.44 in Chloroform, Methanol und Eisessig 5:2:2.

Beispiel 3

4'-O-[4-(alpha-D-Galactopyranosyloxy)-phenylaminocarbonyl]-etoposid (Verbindung 3)

500 mg (0.68 mmol) 2",3"-Di-O-chloracetyl-etoposid wurde in 50 ml DMF gelöst und mit 0.34 ml (3 äq) Diisopropylethylamin und 0.12 ml (1.5 äq) Diphosgen bei Raumtemperatur versetzt. Die Reaktionsmischung wurde 2 h gerührt und anschließend mit 0,22 ml Diisopropylethylamin und 250 mg (0.9 mmol) 4-(alpha-D-Galactopyranosyloxy)-anilin gelöst in 50 ml DMF versetzt. Der Reaktionsansatz wurde 14 h bei Raumtemperatur gerührt, dann mit Ethylacetat versetzt und dreimal mit Citratpuffer (pH 5) ausgewaschen. Die organische Phase wurde über Natriumsulfat getrocknet und in vacuo eingedampft. Der Rückstand wurde säulenchromatographisch über Kieselgel (50 g) mit Chloroform und Methanol 6:1 gereinigt. Es wurden 460 mg 4'-O-[4-(alpha-D-Galactopyranosyloxy)-phenylaminocarbonyl]- 2",3"-di-O-chloracetyl-etoposid erhalten. Dünnschichtchromatographische Analyse: Rf= 0.46 in Chloroform und Methanol 3:1.
Das erhaltene Konjugat (420 mg) wurde in 80 ml Methanol mit 3.0 g Ionenaustauscher Dowex 1x8 entblockiert, wie im Beispiel 1 beschrieben. Es wurden 250 mg Titelverbindung erhalten. Dünnschichtchromatographische Analyse: Rf= 0.41 in Chloroform, Methanol und Eisessig 5:2:2.

Beispiel 4

4'-O-[4-(beta-D-Glucuronyloxy)-phenylaminocarbonyl]-etoposid (Verbindung 4)

500 mg (0.68 mmol) 2",3"-Di-O-chloracetyl-etoposid wurde in 50 ml DMF gelöst und mit 0.34 ml (3 äq) Diisopropylethylamin und 0.12 ml (1.5 äq) Diphosgen bei Raumtemperatur versetzt. Die Reaktionsmischung wurde 2 h gerührt und anschließend mit 0,22 ml Diisopropylethylamin und 250 mg (0.86 mmol) 4-(6-O-Methyl-beta-D-glucuronyloxy)-anilin gelöst in 50 ml DMF versetzt. Der Reaktionsansatz wurde 14 h bei Raumtemperatur gerührt, dann mit Ethylacetat versetzt und dreimal mit Citratpuffer (pH 5) ausgewaschen. Die organische Phase wurde über Natriumsulfat getrocknet und in vacuo eingedampft. Der Rückstand wurde säulenchromatographisch über Kieselgel (50 g) mit Chloroform und Methanol 6:1 gereinigt. Es wurden 460 mg 4'-O-[4-(6-O-Methyl-beta-D-glucuronyloxy)-phenylaminocarbonyl] - -2",3"-di-O-chloracetyl-etoposid erhalten. Dünnschichtchromatographische Analyse: Rf = 0.63 in Chloroform und Methanol 3:1.
Das erhaltene Konjugat (400 mg) wurde in 80 ml Methanol mit 2.0 g Bariumoxid entblockiert. Es wurden 280 mg Titelverbindung erhalten. Dünnschichtchromatographische Analyse: Rf = 0.24 in Chloroform, Methanol und Eisessig 5: 2:2.

Beispiel 5

4'-O-[4-(beta-D-Glucuronyloxy)-3-nitro-benzylaminocarbonyl]-etoposid (Verbindung 5)

500 mg (0.68 mmol) 2",3"-Di-O-chloracetyl-etoposid wurde in 50 ml DMF gelöst und mit 0.34 ml (3 äq) Diisopropylethylamin und 0.12 ml (1.5 äq) Diphosgen bei Raumtemperatur versetzt. Die Reaktionsmischung wurde 2 h gerührt und anschließend mit 0,22 ml Diisopropylethylamin und 250 mg (0.80 mmol) 4-(6-O-Methyl-beta-D-glucuronyloxy)-3-nitro-benzylamin gelöst in 50 ml DMF versetzt. Der Reaktionsansatz wurde 14 h bei Raumtemperatur gerührt, dann mit Ethylacetat versetzt und dreimal mit Citratpuffer (pH 5) ausgewaschen. Die organische Phase wurde über Natriumsulfat getrocknet und in vacuo eingedampft. Der Rückstand wurde säulenchromatographisch über Kieselgel (50 g) mit Chloroform und Methanol 6:1 gereinigt. Es wurden 456 mg 4'-O-[4-(6-O-Methyl-beta-D-glucuronyloxy)-3-nitro-benzyl-aminocarbonyl]-2",3"-di-O-chloracetyl-etoposid erhalten. Dünnschichtchromatographische Analyse: Rf = 0.64 in Chloroform und Methanol 3:1. Das erhaltene Konjugat (400 mg) wurde in 80 ml Methanol mit 2.0 g Bariumoxid entblockiert. Es wurden 320 mg Titelverbindung erhalten. Dünnschichtchromatographische Analyse: Rf= 0.27 in Chloroform, Methanol und Eisessig 5:2:2..

Beispiel 6

4'-O-[4-(beta-D-Glucuronyloxy)-3-chlor-benzylaminocarbonyl]-etoposid (Verbindung 6)

500 mg (0.68 mmol) 2",3"-Di-O-chloracetyl-etoposid wurde in 50 ml DMF gelöst und mit 0.34 ml (3 äq) Diisopropylethylamin und 0.12 ml (1.5 äq) Diphosgen bei Raumtemperatur versetzt. Die Reaktionsmischung wurde 2 h gerührt und anschließend mit 0,22 ml Diisopropylethylamin und 250 mg (0.80 mmol) 4-(6-O-Methyl-beta-D-glucuronyloxy)-3-chlor-benzylamin gelöst in 50 ml DMF versetzt. Der Reaktionsansatz wurde 14 h bei Raumtemperatur gerührt, dann mit Ethylacetat versetzt und dreimal mit Citratpuffer (pH 5) ausgewaschen. Die organische Phase wurde über Natriumsulfat getrocknet und in vacuo eingedampft. Der Rückstand wurde säulenchromatographisch über Kieselgel (50 g) mit Chloroform und Methanol 6:1 gereinigt. Es wurden 430 mg 4'-O-[4-(6-O-Methyl-beta-D-glucuronyloxy)-3-chlor-benzyl-aminocarbonyl]-2",3"-di-O-chloracetyl-etoposid erhalten. Dünnschichtchromatographische Analyse: Rf= 0.68 in Chloroform und Methanol 3:1. Das erhaltene Konjugat (400 mg) wurde in 80 ml Methanol mit 2.0 g Bariumoxid entblockiert. Es wurden 340 mg Titelverbindung erhalten. Dünnschichtchromatographische Analyse: Rf = 0.29 in Chloroform, Methanol und Eisessig 5:2:2.

Beispiel 7

1-N-[4-(beta-D-Glucuronyloxy)-benzyloxycarbonyl]-mitomycin C (Verbindung 7)

500 mg (1.13 mmol) 4-(6-Methyl-2,3,4-tri-O-acetyl-beta-D-glucuronyloxy)-benzyl-alkohol wurden in 20 ml Toluol gelöst und mit 440 mg (3 äq) Diisopropylethylamin und 315 mg (1.5 äq) Diphosgen versetzt. Die Mischung wurde 1 h bei Raumtemperatur gerührt. Anschließend wurden 680 mg (1.8 äq) Mitomycin C und 290 mg (2 äq) Diisopropylethylamin gelöst in 50 ml DMF zugegeben. Die Reaktionsmischung wurde 14 h gerührt, dann mit Ethylacetat versetzt und mit Citratpuffer ausgewaschen. Die organische Phase wurde über Natriumsulfat getrocknet und in vacuo eingedampft. Rohausbeute: 651 mg (72%). Das Konjugat (600 mg) wurde in 30 ml Chloroform und Methanol 2:1 gelöst und mit 250 mg Bariumoxid versetzt. Nach 4 h Rühren wurde der Ansatz abfiltriert und das Filtrat wurde eingedampft. Der Rückstand wurde säulenchromatographisch über Sephadex mit Methanol und Wasser gereinigt. Ausbeute der Titelverbindung: 335 mg (75%).

Beispiel 8

14-O-[4-(beta-D-Glucuronyloxy)-3-nitro-benzylaminocarbonyl]-doxorubicin (Verbindung 8)

400 mg (0,52 mmol) 3'-N-Fluorenylmethyloxycarbonyl-doxorubicin wurden in 20 ml Toluol gelöst und mit 200 mg (3 äq) Diisopropylethylamin und 144 mg (1.5 äq) Diphosgen versetzt. Die Reaktionsmischung wurde 1 h bei Raumtemperatur gerührt und mit 294 mg (1.8 äq) 4-(6-O-Methyl-beta-D-glucuronyloxy)-3-nitro-benzylamin und 134 mg (2 äq) Diisopropylethylamin gelöst in 50 ml DMF versetzt. Die Mischung wurde 14 h gerührt, dann mit Ethylacetat versetzt und mit Citratpuffer (pH 5) ausgewaschen. Die organische Phase wurde über Natriumsulfat getrocknet und in vacuo eingeengt. Das Rohprodukt wurde säulenchromatographisch über Kieselgel gereinigt. Ausbeute: 370 mg (65%). Die geschützte Zwischenverbindung (300 mg) wurde in 20 ml THF gelöst und mit 1.0 ml Piperidin versetzt. Nach 14 h Rühren wurde der Ansatz in vacuo eingedampft und mit Toluol codestilliert. Der Rückstand wurde in 20 ml Methanol gelöst und mit 250 mg Bariumoxid versetzt. Nach 5 h Rühren wurde die Reaktionsmischung abfiltriert und das Filtrat wurde in vacuo eingedampft. Der Rückstand wurde säulenchromatographisch über Sephadex mit Methanol und Wasser gereinigt. Ausbeute der Titelverbindung: 140 mg (56%).

Beispiel 9

4-O-[4-(beta-D-Glucuronyloxy)-benzylaminocarbonyl]-4-hydroxy-1-N-(bis-2-chlorethyl)-anilin

(Verbindung 9)

400 mg (171 mmol) 4-Hydroxy-1-N-(bis-2-chlorethyl)-anilin wurden in 20 ml Toluol gelöst und mit 950 mg Diisopropylethylamin und 500 mg (1.5 äq) Diphosgen versetzt. Nach 1 h Rühren wurden zu der Reaktionsmischung 960 mg (1.8 äq) 4-(6-O-Methyl-beta-D-glucuronyloxy)-benzylamin und 0.63 ml Diisopropylethylamin gelöst in 50 ml DMF zugegeben. Der Reaktionsansatz wurde 14 gerührt, dann mit Ethylacetat versetzt und mit Citratpuffer ausgewaschen. Die organische Phase wurde über Natriumsulfat getrocknet und in vacuo eingedampft. Der Rückstand (550 mg) wurde in Methanol gelöst und mit 400 mg Bariumoxid versetzt. Die Reaktionsmischung wurde 4 h gerührt, abfiltriert und in vacuo eingedampft. Der Rückstand wurde säulenchromatographisch über Sephadex gereinigt. Ausbeute der Titelverbindung: 420 mg.

Beispiel 10

4-O-[4-(beta-D-Glucuronyloxy)-benzylaminocarbonyl]-terfenadin

(Verbindung 10)

Ausgehend von Terfenadin und 4-(6-O-Methyl-beta-D-glucuronyloxy)-benzylamin wurde die Titelverbindung, wie im Beispiel 9 beschrieben, hergestellt.

Beispiel 11

3'-O-[4-(beta-D-Glucuronyloxy)-benrylaminocarbonyl]-terbutalin

(Verbindung 11)

Ausgehend von Terbutalin und 4-(6-O-Methyl-beta-D-glucuronyloxy)-benzylamin wurde die Titelverbindung, wie im Beispiel 9 beschrieben, hergestellt.

Beispiel 12

3'-O-[4-(beta-D-Glucuronyloxy)-benrylaminocarbonyl]-fenoterol

(Verbindung 12)

Ausgehend von Fenoterol und 4-(6-O-Methyl-beta-D-glucuronyloxy)-benzylamin wurde die Titelverbindung, wie im Beispiel 9 beschrieben, hergestellt.

Beispiel 13

1"-O-[4-(beta-D-Glucuronyloxy)-benzylaminocarbonyl]-salbutamol

(Verbindung 13)

Ausgehend von Salbutamol und 4-(6-O-Methyl-beta-D-glucuronyloxy)-benzylamin wurde die Titelverbindung, wie im Beispiel 9 beschrieben, hergestellt.

Beispiel 14

3-O-[4-(beta-D-Glucuronyloacy)-benrylaminocarbonyl]-muscarin

(Verbindung 14)

Ausgehend von Muscarin und 4-(6-O-Methyl-beta-D-glucuronyloxy)-benzylamin wurde die Titelverbindung,

wie im Beispiel 9 beschrieben, hergestellt.

Beispiel 15

4'-O-[4-(beta-D-Glucuronyloxy)-benzylaminocarbonyl]-oxyphenbutazon (Verbindung 15)

Ausgehend von Oxyphenbutazon und 4-(6-O-Methyl-beta-D-glucuronyloxy)-benzylamin wurde die Titelverbindung, wie im Beispiel 9 beschrieben, hergestellt.

Beispiel 16

2-O-[4-(beta-D-Glucuronyloxy)-benzylaminocarbonyl]-salicylsäure (Verbindung 16)

Ausgehend von Salicylsäuremethylester und 4-(6-O-Methyl-beta-D-glucuronyloxy)-benzylamin wurde die Titelverbindung, wie im Beispiel 9 beschrieben, hergestellt.

Beispiel 17

N-[4-(beta-D-Glucuronyloxy)-benzyloxycarbonyl]-diclofenac

(Verbindung 17)

Ausgehend von 4-(6-Methyl-2,3,4-tri-O-acetyl-beta-D-glucuronyloxy)-benzyl-alkohol und Diclofenac wurde die Titelverbindung, wie im Beispiel 7 beschrieben, hergestellt.

Beispiel 18

N-[4-(beta-D-Glucuronyloxy)-benzyloxycarbonyl]-flufenaminsäure

(Verbindung 18)

Ausgehend von 4-(6-Methyl-2,3,4-tri-O-acetyl-beta-D-glucuronyloxy)-benzyl-alkohol und Flufenaminsäure wurde die Titelverbindung, wie im Beispiel 7 beschrieben, hergestellt.

Beispiel 19

4-N-[4-(beta-D-Glucuronyloxy)-benzyloxycarbonyl]-4-methyl- aminophenazon

(Verbindung 19)

Ausgehend von 4-(6-Methyl-2,3,4-tri-O-acetyl-beta-D-glucuronyloxy)-benzyl-alkohol und 4-Methylaminophenazon wurde die Titelverbindung, wie im Beispiel 7 beschrieben, hergestellt.

Beispiel 20

7-N-[4-(beta-D-Glucuronyloxy)-benzyloxycarbonyl]-theophyllin (Verbindung 20)

Ausgehend von 4-(6-Methyl-2,3,4-tri-O-acetyl-beta-D-glucuronyloxy)-benzyl-alkohol und Theophyllin wurde die Titelverbindung, wie im Beispiel 7 beschrieben, hergestellt.

Beispiel 21

1-N-[4-(beta-D-Glucuronyloxy)-benzyloxycarbonyl]-nifedipin (Verbindung 21)

Ausgehend von 4-(6-Methyl-2,3,4-tri-O-acetyl-beta-D-glucuronyloxy)-benzyl-alkohol und Nifedipin wurde die Titelverbindung, wie im Beispiel 7 beschrieben, hergestellt.

Beispiel 22

[4-(β-D-glucuronyloxy)-3-nitrobenzyl]-2-[1-cyano-1-(N-4-trifluormethylphenyl)carbamoyl]propen-1-yl-carbonat (Verbindung 22)

Der Chlorameisensäureester (4-[2,3,4-tri-O-Acetyl-β-D-glucopyranosyl)methyluronat]-3-nitro-benzyloxycarbonylchlorid) läßt sich nach bekannten Methoden herstellen, z.B. aus Methyl-(4-hydroxymethyl-2-nitrophenyl-2,3,4-tri-O-acetyl-β-D-glucopyranosid)uronat und Phosgen. Methyl-(4-hydroxymethyl-2-nitrophenyl-2,3,4-tri-O-acetyl-β-D-glucopyranosid)uronat läßt sich aus Methyl-(2,3,4-tri-O-acetyl-β-D-glucopyranosyl)uronatbromid durch Umsetzung mit 2-Hydroxy-5-nitrobenzaldehyd und anschliessende Reduktion gewinnen.
2-Cyano-3-hydroxy-N-(trifluormethylphenyl)crotonsäureamid ist der aktive Metabolit von Leflunomid, das Hydroxycrotonsäureamid-Derivat 2 (WO 91/17748). Es wird gemäß dort beschriebenem Beispiel 4B durch alkalische Ringöffnung von Leflunomid gewonnen.
5.5 g (0.091 Mol) 4-[(2,3,4-tri-O-Acetyl-β-D-glucopyranosyl) methyluronat]-3-nitrobenzyloxycarbonylchlorid und 2.7 g (0.01 Mol) 2-Cyano-3-hydroxy-N-trifluormethylphenyl)crotonsäureamid werden in 80 ml Acetonitril gelöst. Nach Zugabe von 1.7 g (0.01 Mol) AgNO3 wird unter Rühren 3 Stunden auf 60° C erhitzt. Das nach dem Abkühlen und Abfiltrieren erhaltene Filtrat wird unter vermindertem Druck bis zur Trockne eingeengt. Das Rohprodukt wurde in 200 ml Chloroform/ Methanol 2:1 (v:v) gelöst und mit 1.5 g Bariumoxid versetzt. Der Ansatz wurde 5 h gerührt und das nach dem Abfiltrieren erhaltene Filtrat bis zur Trockne eingedampft. Nach säulenchromatographischer Reinigung erhält man so die Verbindung 22.

Beispiel 23

N-[N-(alpha-D-galactopyranosyloxycarbonyl)-4-hydroxymethyloxycarbonyl anilin]-doxorubicin (Verbindung 23)

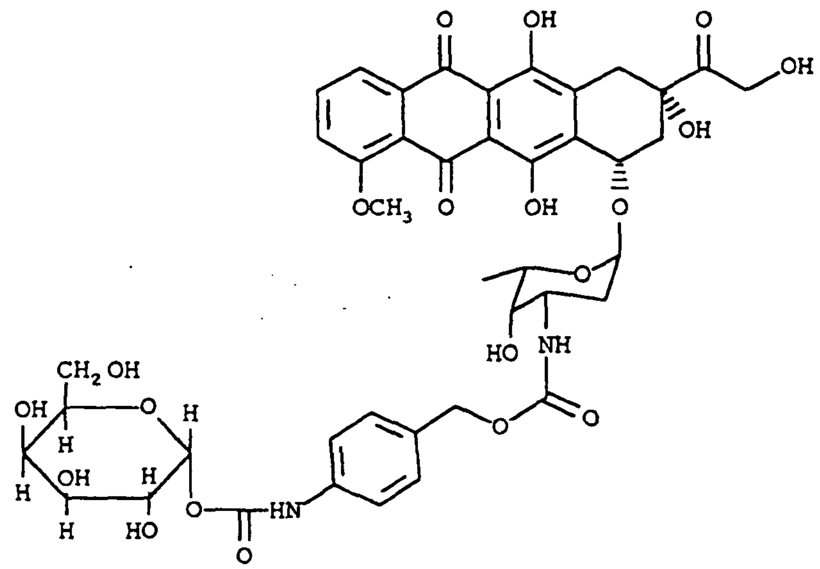

*2,3,4, 6-tetra-O-acetyl-D-galactopyranosid (2)*

Zu einer Lösung von Penta-O-acetyl-D-galactose (10 g, 25.6 mmol) in DMF wurde Hydrazinacetat (3.5 g, 38 mmol) hinzugegeben. Die Mischung wurde bei 80° C 15 Minuten lang erhitzt. Die abgekühlte Lösung wurde mit Wasser (100 ml) verdünnt und mit Ethylacetat extrahiert. Die organische Phase wurde mit Salzwasser gewaschen, getrocknet ($MgSO_4$) und abgedampft. Aus dem Rückstand erhielt man nach Flash-Chromatographie (Cyclohexan : Ethylacetat, 3: 2, v/v) 4.95 g (55 %) von Verbindung 2 als Feststoff.

Verbindung 2: $C_{14}H_{20}O_{10}$, Schmelzpunkt: 104°C

(fortgesetzt)

$[\alpha]_D^{20}$ + 95° C (C1, $CHCl_3$)

*N-[2,3,4,6-tetra-O-azetyl-a-D-galactopyranosylozy]-p-toluidin (3)*

Zu einer Lösung von Verbindung 2 (3.48 g, 10 mmol) in DMF (50 ml) wurde P-Tolylisocyanat (4.4 g, 33 mmol) zugegeben. Nach 15 hrs Rühren bei 40° C wurde die Reaktionsmischung mit $H_2O$ (120 ml) verdünnt und mit Ethylacetat extrahiert. Die organische Phase wurde zuerst mit $H_2O$ (2 x 50 ml), dann mit Salzlösung gewaschen, getrocknet ($MgSO_4$) und dann unter reduziertem Druck abgedampft. Nach Flash-Chromatographie (Cyclohexan : Ethylacetat, 2:1, v/v) wurde Verbindung 3 (2.2 g, 45 %) erhalten.

| | |
|---|---|
| Verbindung 3: | $C_{22}H_{27}O_{11}N$, Schmelzpunkt: 93° C |
| | $[\alpha]_D^{20}$ + 84° C (C1, $CHCl_3$) |

*N-(2,3,4,6-tetra-O-acetyl-α-D-galactopyranosloxycarbonyl)4-formylanilin (6) und N-(2,3,4,6-tetra-O-acetyl-α-D-galactopyranosylorycarbonyl)4-hydroxymethylanilin (7)*

Zu einer Lösung von Verbindung 3 (1 g) in $CCl_4$ (60 ml) wurden N-bromosuccinimid (420 mg, 1.1 eq) und 120 mg Benzoylperoxid hinzugegeben. Nach 4 hrs Rühren unter Rückfluß wurde die abgekühlte Mischung filtriert und das Filtrat mit $H_2O$, dann mit Salzlösung gewaschen und getrocknet ($MgSO_4$). Nach Abdampfen unter reduziertem Druck wurde ein Rückstand von 1 g erhalten, der mit Flash-Chromatographie gereinigt wurde und 800 mg einer Mischung von Monobrom- und Dibrom-Verbindungen ergab. Diese Mischung wurde direkt weiterverwendet. 800 mg der Mischung wurden in Aceton (14 ml) gelöst und eine wässrige Lösung von Silbernitrat (0.1 N, 14 ml) wurde hinzugegeben. Die Reaktionsmischung wurde 3 hrs bei Raumtemperatur gerührt. Nach Filtration über Celit wurde das Filtrat unter reduziertem Druck eingedampft und die verbleibende wässrige Phase mit $CH_2Cl_2$ (Dichlormethan) extrahiert. Die organische Phase wurde mit $H_2O$ und Salzlösung gewaschen, getrocknet ($MgSO_4$) und unter reduziertem Druck eingedampft. Der erhaltene Rückstand wurde mit Flash-Chromatographie (Cyclohexan : Aceton, 2:1, v/v) gereinigt und ergab 120 mg von Verbindung 6 und 260 mg von Verbindung 7.

| | |
|---|---|
| Verbindung 6: | $C_{22}H_{25}O_{12}N$, Schmelzpunkt: 81°C |
| | $[\alpha]_D^{20}$ + 109° (C1, $CHCl_3$) |
| Verbindung 7: | $C_{22}H_{27}O_{12}H$, Schmelzpunkt: 85°C |
| | $[\alpha]_D^{20}$ + 129° (C1, $CHCl_3$) |

*N-(2,3,4,6-tetra-O-acetyl-α-D-galactopyranosyloxycarbonyl)-[(4-nitrophenyloxycarbonyl)-4-hydroxymethylanilin] (8)*

Zu einer Lösung von Verbindung 7 (300 mg) in $CH_2Cl_2$ (30 ml) wurde P-nitrophenylchloroformiat (360 mg) und Pyridin (0.18 ml) hinzugegeben. Nach 3 hrs Rühren bei Raumtemperatur wurde die Reaktionsmischung mit $H_2O$ (50 ml) verdünnt und extrahiert. Die vereinigten organischen Phasen wurden mit $H_2O$ und Salzlösung gewaschen und getrocknet ($MgSO_4$). Der nach Eindampfen unter reduziertem Druck erhaltene Rest wurde mit Flash-Chromatographie (Cyclohexan-Aceton, 2:1, v/v) gereinigt und ergab 380 mg (51 %) von Verbindung 8.

| | |
|---|---|
| Verbindung 8: | $C_{29}H_{30}O_{16}N_2$, Schmelzpunkt: 92° C |
| | $[\alpha]_D^{20}$ + 81° (C1, $CHCl_3$) |

*N-[N-(2,3,4,6-tetra-O-acetyl-α-D-galactopyranosylorycarbonyl)4-hydroxymethyl- oxycarbonylanilin] doxorubicin (9)*

Zu einer Lösung von Verbindung 8 (100 mg) in DMF wurden 100 mg Doxorubicin und 80 μl Triethylamin gegeben. Die Reaktionsmischung wurde 6 h bei Raumtemperatur gerührt. Nach Eindampfen unter reduziertem Druck (1 mm, Δ = 50° C) wurde der erhaltene Rest mit Flash-Chromatographie gereinigt und ergab 80 mg (50 %) von Verbindung 9.

| | |
|---|---|
| Verbindung 9: | $C_{50}O_{24}H_{54}N_2$, Schmelzpunkt: 142° C |
| | $[\alpha]D^{20}$ + 196° (C1, $CHCl_3$) |

*Verbindung 23*

Zu einer Lösung von Verbindung 9 (100 mg) in MeOH (Methanol) (30 ml) wurden 20 mg Natriummethanolat hinzugegeben. Die Reaktionsmischung wurde 3 hrs gerührt und mit Amberlite IRC-50 ($H^+$) neutralisiert. Nach Filtration wurde das Filtrat unter reduziertem Druck eingeengt und ergab einen festen Rückstand. Flash-Chromatographie auf Silica (Acetonitril : Wasser, 9:1, v/v) dieses Rückstandes ergab 67 mg (80 %) von Verbindung 23.

Verbindung 23: $C_{42}H_{46}O_{20}N_2$, Schmelzpunkt: 136° C
$[\alpha]_D^{20}$ + 225° (C 0.1, ETOH)

1H NMR (270 MHz, $D_2O$): δ 1.3 (d, J = 6, 3H, Me-6); 1.9 und 2.2 (AB, 2H, $CH_2$-8, J = 9); 2.8 und 2.9 (AB, 2H, $CH_2$-2, J = 20); 3.8 (S, 3H, $OCH_3$); 5.3 (m, 1H, H-1'); 6.0 (d, 1H, J = 3.5); 7.1 (d, 1H, J = 8, H-3); 7.3 (d, J = 8, 1H, H-2); 7.5 (d, 1H, J = 8, H-1); 7.8 (m, H-arom).

Beispiel 24

9-O-[4-(beta-D-glucuronyloxy)-3-chlorobenzyloxycarbonyl]-quinin (Verbindung 24)

*4-β-D-methylglucuronyloxy-3-chlorobenzaldehyd (1)*

Eine Lösung von 4-(2,3,4-tri-O-acetyl-β-D-methylglucuronyloxy)-3-chlorobenzaldehyd (3.05 g, 6.45 mmol) in 0.1 N-methanolischer Natriummethanolatlösung (50 ml) wurde bei 0° C 30 Minuten lang gerührt. Die Reaktionsmischung wurde durch die Zugabe von Amberlite IRC 120 $H^+$ Ionenaustauscher neutralisiert, filtriert und unter reduziertem Druck eingeengt und ergab 4-β-D-methylglucuronyloxy-3-chloröbenzaldehyd (1) als Schaum (2.17 g, 97 %).
$C_{14}H_{15}ClO_8$ M = 346.5
IR (KBr) ν cm -1: 3400, 3030, 2975, 1375, 1040, 835.
$^1H$ NMR (300 MHz, $(CD_3)_2SO$) : 3.25-4.25 (4H, m), 3.70 (3H, s), 5.05 (1H, d, J = 7 Hz), 7.28 (1H, d, J = 8 Hz), 7.75 (1% dd, J = 8 Hz, J' = 2 Hz), 7.90 (1H, d, J = 2 Hz), 9.85 (1H, s).
Ms (DCI/$NH_3$) : m/z : 364/366 $(M+NH_4)^+$.

*4-[2,3,4-tri-O-(4-methoxybenzyl)-β-D-methylglucuronyloxy]-3-chlorobenzaldehyd (2)*

Eine Lösung von 4-β-D-methylglucuronyloxy-3-chlorobenzaldehyd (2.10 g, 6.06 mmol) in trockenem Dimethylformamid (20 ml) wurde langsam zu einer Suspension von Natriumhydrid (2 g) in 20 ml trockenem Dimethylformamid hinzugegeben. 4-Methoxybenzylchlorid (3.2 ml, 23.5 mmol) wurden tropfenweise über 30 Minuten hinweg hinzugegeben, und die Reaktionsmischung wurde gekühlt, um die Temperatur ungefähr bei 20° C zu halten. Die Lösung wurde weitere 18 hrs gerührt und durch die Zugabe von Methanol (5 ml) aufgearbeitet. Nach 1 h wurde die Lösung auf Eis gegossen und auf pH 8 mit 1M Salzsäure eingestellt. Die wässrige Phase wurde mit Ethylacetat (2 x 50 ml) extrahiert. Nach Waschen mit $H_2O$ (2 x 50 ml) wurde die organische Phase zur Trockne eingedampft. Die Reinigung des Rückstandes mit Säulenchromatographie auf Silica Gel 60 H [Hexan : Ethylacetat (8:2 v/v)] ergab 4-[2,3,4-tri-O-(4-methoxybenzyl)-β-D-methylglucuronyloxy]-3-chlorobenzaldehyd (2) als einen farblosen Schaum (1.97 g, 46 %). $C_{38}H_{39}CLO_{11}$, M = 706.5
IR (KBr) ν $cm^{-1}$: 3040, 2945, 1725, 1505, 1375, 1130, 1040, 825.
$^1H$ NMR (300 MHz, $CDCl_3$) : 3.30-4.50 (4H, m), 3.73 (3H, s), 3.85 (3H, s), 3.88 (3H, s), 3.92 (3H, s), 4.50-4.90 (6H, m), 5.12 (1H, d, J = 7 Hz), 6.90-7.80 (15H, m), 10.02 (1H, s). MS (DCI/$NH_3$) ; m/z : 722/724 $(M+NH_4)^+$.

*2-Chloro-4-hydroxymethylphenyl-2,3,4-tri-O-(4-methoxybenzyl)-β-D-methylglucuronid (3)*

Eine Lösung des Aldehyds 2 (1.57 g, 2.22 mmol) in Methanol (30 ml) wurde mit Natriumborhydrid (0.7 g) behandelt und die Reaktionsmischung bei 0° C 90 Minuten lang gerührt. Die mit Wasser abgestoppte Reaktion wurde dann mit Dichlormethan (3 x 20 ml) extrahiert. Die Reinigung über Säulenchromatographie über Silica Gel 60 H [Hexan : Ethylacetat (7:3 v/v)] ergab 2-chloro-4-hydroxymethylphenyl-2,3,4-tri-O-(4-methoxybenzyl)-β-D-methylglucuronide (3) als amorphen Feststoff(1.28 g, 81 %).
$C_{38}H_{41}ClO_{11}$, M = 708.5
IR (KBr) ν cm-1 : 3420, 3030, 2935, 1725, 1510, 1510, 1375, 1230, 1040, 825.
$^1H$ NMR (300 MHz, $CDCl_3$) : 3.25-4.50 (4H, m), 3.69 (3H, s), 3.85 (3H, s), 3.87 (3H, s), 3.91 (3H, s), 4.50-4.80 (8H, m), 5.08 (1H, d, J = 7 Hz), 6.85-7.90 (15H, m).
MS (DCI/$NH_3$) : m/z : 724/726 $(M+NH_4)^+$.

*4-[2,3,4-tri-O-(4-methoxybenzyl)-*β*-D-methylglucuronyloxy]-3-chloro*-benzyl-4-nitrophenyl-*carbonat (4)*

Der Alkohol 3 (0.25 g, 0.35 mmol) wurde in Ethylacetat (1.5 ml) und Pyridin (0.15 ? ml) gelöst. 4-Nitrophenylchloroformiat (0.30 g, 1.48 mmol) wurde hinzugegeben und die Reaktionsmischung über Nacht gerührt. Die Lösungsmittel wurden unter reduziertem Druck entfernt. Die Reinigung des Rückstandes durch Säulenchromatographie über Silica Gel 60 H [Hexan : Ethylacetat (7:3 v/v)] ergab 4-[2,3,4-tri-O-(4-methoxybenzyl)-β-D-methylglucuronyloxy]-3-chloro-benzyl-4-nitrophenylcarbonat(4) (0.16 g , 52%).

$C_{45}H_{44}ClNO_{15}$, M = 873.5

IR (KBr) ν cm-1 : 3055, 2930, 1735, 1515, 1370, 1320, 1230, 1040, 830.

$^1$H NMR (300 MHz, $CDCl_3$) : 3.25-4.50 (4H, m), 3.71 (3H, s), 3.82 (3H, s), 3.87 (3H, s), 3.91 (3H, s), 4.45-4.85 (6H, m), 5.05 (1H, d, J = 7 Hz), 5.15 (2H, s), 6.80-8.40 (19H, m).

MS ($DCl/NH_3$) : 891/893 $(M+NH_4)^+$.

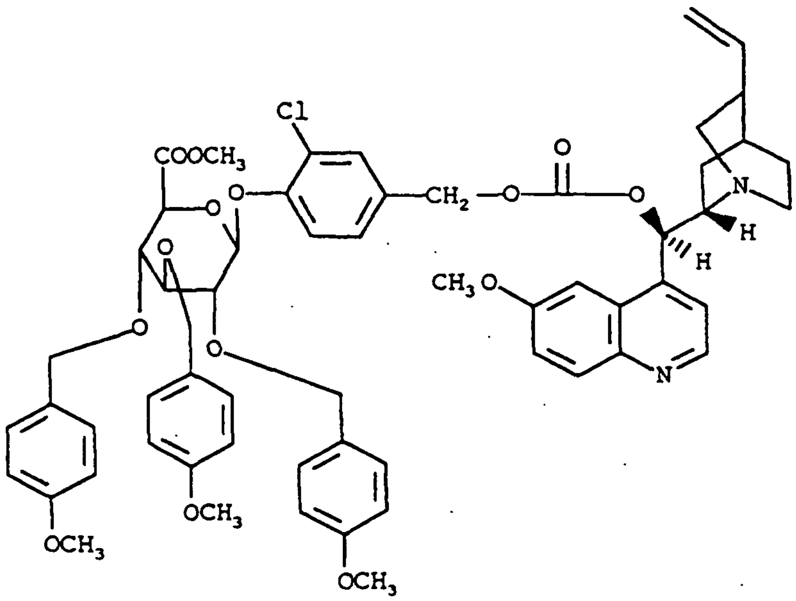

Zu einer gerührten Lösung von 4 (0.15 g, 0.17 mmol) in Dichlormethan (20 ml) und Triethylamin (188 μl) wurde Quinin hinzugegeben (50 mg, 0.15 mmol). Die Mischung wurde für 80 hrs bei Raumtemperatur gehalten. Die Lösungsmittel wurden unter reduziertem Druck abgedampft. Die Reinigung des Rückstandes durch Säulenchromatographie und Silica Gel 60 H [Dichlormethan : Methanol (95:5 v/v)] ergab 5 als farblosen Schaum (0.057 g, 36 %).

$C_{59}H_{63}ClN_2O_{14}$, M = 1058.5

IR (KBr) ν cm-1 : 3050, 2940, 1740, 1520, 1370, 1320, 1230, 1010, 835, 810.

$^1$H NMR (300 MHz, $CDCl_3$) : 1.80-4.40 (15H, m), 3.72 (3H, s), 3.85 (3H, s)., 3.93 (6H, s), 3.95 (3H, s), 4.45-4.85 (6H, m), 5.10 (1H, d, J = 7 Hz), 5.15 (2H, s), 5.30 (2H, m), 5.72 (1H, m), 6.30 (1H, d, J = 7 Hz), 6.80-8.40 (19H, m), 8.75 (1H, d, J = 5 Hz)

MS ($DCl/NH_3$) : 1076/1078 $(M+NH_4)^+$.

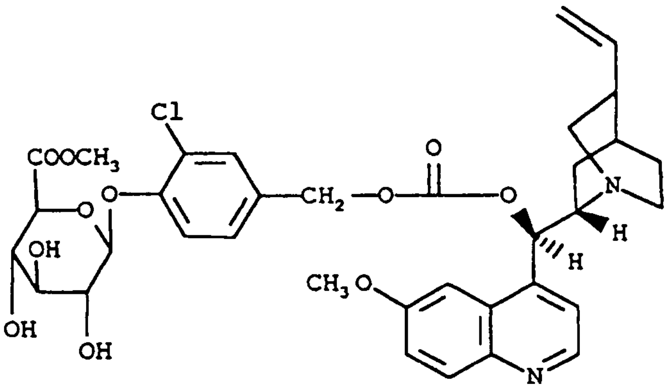

Zu einer gerührten Lösung von 5 (0.050 g, 0.05 mmol), die in Dichlormethan (10 ml) und Wasser (0.5 ml), gelöst war, wurden bei 5° C 2,3-dichloro-5,6-dicyano-1,4-benzoquinone (0.017 g, 0.075 mmol) hinzugegeben. Nach 1 h wurde eine gesättigte wässrige Natriumhydrogencarbonatlösung (10 ml) hinzugegeben und die Mischung wurde schnell mit Dichlormethan (3x 15 ml) extrahiert. Die organische Phase wurde mit Wasser gewaschen, über Natriumsulfat getrocknet und reduziertem Druck abgedampft. Der Rückstand wurde auf einer Silica Gel 60 H Säule [Dichlormethan : Methanol (80:20 v/v)] chromatographiert und ergab 6 als einen amorphen Reststoff(0.023 g, 66 %).
$C_{35}H_{39}ClN_2O_{11}$, M = 698.5
IR(KBr) ν cm-1 : 3420, 3040, 2940, 1735, 1530, 1375, 1320, 1235, 1020, 830.
$^1$HNMR (300 MHz, $(CD_3)_2SO$) : 1.80-4.30 (15H, m), 3.70 (3H, s), 3.94 (3H, s), 5.12 (1H, d, J = 7 Hz), 5.17 (2H, s), 5.30 (2H, m), 5.71 (1H, m), 6.34 (1H, d, J = 7 Hz), 7.10-8.00 (6H, m), 8.77 (1H, d, J = 5 Hz).
MS (FAB, Matrix : Nitrobenzyl Alkohol) : 699/701 $(m+H)^+$.

*Verbindung 24*

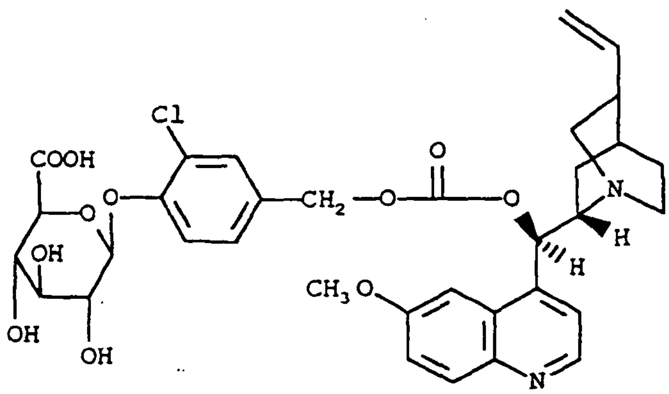

Zu einer Lösung von 6 (0.020 g, 0.03 mmol) in wässrigem Phosphatpuffer (pH 8) (24 ml) wurde eine Schweineleber-Esteraselösung (Sigma # E-3128) (0.6 ml) und Aceton (12 ml) hinzugegeben. Die Mischung wurde für 4 hrs bei 37° C gehalten und unter reduziertem Druck eingeengt. Die Reinigung des Rückstandes durch Säulenchromatographie und Silica Gel 60 H [Acetonitril : Wasser (95:5 v/v)] ergab Verbindung 24 als einen farblosen Feststoff (0.011 g, 56 %).
$C_{34}H_{37}ClN_2O_{11}$, M = 684.5
IR (KBr) ν cm-1 : 3450, 3060, 2950, 1730, 1520, 1370, 1320, 1240, 1020, 820.
$^1$H-NMR (300 MHz, $(CD_3)_2SO$) : 1.80-4.30 (15H, m), 3.94 (3H, s)., 5.10 (1H, d, J = 7 Hz), 5.22 (2H, s), 5.32 (2H, m), 5.70 (1H, m), 6.32 (1H, d, J = 7 Hz), 7.10-8.00 (6H, m), 8.79 (1H, d, J = 5 Hz).
MS (FAB, Matrix : Nitrobenzyl Alkohol) : 685/687 $(M+H)^+$.

Beispiel 25

Methyl- 18-O-[3,5-dimethoxy-4-[4-(beta-D-glucuronyloxy)-3-chlorobenzyloxycarbonyl]benzoyl]-reserpat (Verbindung 25)

*4-β-D-glucuronyloxy-3-chlorobenzaldehyd (8)*

Zu einer Lösung von 4-β-D-methylglucuronyloxy-3-chlorobenzaldehyd (1) (3.52 g, 10.1 mmol) in Tetrahydrofuran (60 ml) und Wasser (40 ml) wurde tropfenweise 2M wässrige Natriumhydroxidlösung (10 ml) über 30 Minuten hinzugegeben. Die Mischung wurde 2 hrs gerührt, durch Zugabe von Amberlite IRC 50S H+ Ionenaustauscher neutralisiert, filtriert und zur Trockne eingedampft. Diese ergab 4-β-D-glucuronyloxy-3-chlorobenzaldehyd (8) als einen farblosen Schaum (2.62 g, 78 %).
$C_{13}H_{13}ClO_8$, M= 332.5
IR (KBr) ν cm-1 : 3400, 3050, 2940, 1735, 1330, 1040, 830.
$^1$H NMR (300 MHz, $(CD_3)_2SO$) : 3.25-4.25 (4H, m), 5.05 (1H, d, J = 7 Hz), 7.31 (1H, d, J = 8 Hz), 7.77 (1H, dd, J = 8 Hz, J' = 2 Hz), 7.92 (1H, d, J = 2 Hz), 9.82 (1H, s), 11.85 (1H, br. s).
MS (DCl/$NH_3$) ; m/z : 350/352 (M+NH4)$^+$.

*4-(2,3,4-tri-O-benzyl)-β-D-benzylglucuronyloxy-3-chlorobenzaldehyd (9)*

Eine Lösung von 9 (2.55 g, 7.67 mmol) in trockenem Dimethylformamid (20 ml) wurde langsam zu einer Suspension von Natriumhydrid (2 g) in 20 ml trockenem Dimethylformamid gegeben. Benzylbromid (5.0 ml, 42 mmol) wurde tropfenweise über 30 Minuten unter Kühlung hinzugegeben, so daß die Reaktionstemperatur etwa 20° C betrug. Die Lösung wurde weitere 18 hrs gerührt und durch die Zugabe von Methanol (5 ml) aufgearbeitet. Nach 1 h wurde die Lösung in Wasser gegossen und mit 1 M Salzsäure auf pH 8 gebracht. Die wässrige Phase wurde mit Ethylacetat (2 x 50 ml) extrahiert. Nach Waschen mit Wasser (2 x 50 ml) wurde die organische Phase zur Trockne eingedampft. Der Rückstand wurde durch Säulenchromatographie auf Silica Gel 60 H [Hexan : Ethylacetat (85:15 v/v)] gereinigt und ergab 4-(2,3,4-tri-O-benzyl)-β-D-benzylglucuronyl-oxy-3-chlorobenzaldehyd (9) als farblosen Schaum (2.04 g, 38 %).
$C_{41}H_{37}ClO_8$, M = 692.5
IR (KBr) ν cm-1 : 3050, 2930, 1730, 1520, 1370, 1330, 1230, 1040, 825
$^1$H NMR (300 MHz, $CDCl_3$) : 340-4.50 (4H, m), 4.50-5.00 (8H, m), 5.08 (1H, d, j = 7 Hz), 6.90-7.70 (23H, m), 9.93 (1H, s).
MS (DCl/$NH_3$) : m/z : 710/712 (M+$NH_4$)$^+$.

*2-Chloro-4-hydroxymethylphenyl-2,3,4-tri-O-benzyl-β-D-benzylglucuronid (10)*

Eine Lösung des Aldehyds 9 (2.00 g, 2.88 mmol) in Methanol (30 ml) wurde mit Natriumborhydrid (0.7 g) behandelt und die Reaktionsmischung 90 Minuten bei 0° C gerührt. Die mit Wasser abgestoppte Reaktion wurde dann mit Dichlormethan (3 x 20 ml) extrahiert. Die Reinigung durch Säulenchromatographie über Silica Gel 60 H [Hexan : Ethylacetat (7:3 v/v)] ergab 2-chloro-4-hydroxymethylphenyl-2,3,4-tri-O-benzyl-β-D-benzylglucuronid (10) als amorphen Feststoff (1.91 g, 95 %).
$C_{41}H_{39}ClOg$, M = 694.5
IR (KBr) ν cm-1 : 3400, 3040, 2940, 1725, 1515, 1370, 1330, 1230, 1040, 825
$^1$H NMR (300 MHz, $CDCl_3$) : 3.40-4.50 (4H, m), 4.50-5.00 (10H, m), 5.12 (1H, d, J = 7 Hz), 6.90-7.75 (23H, m).
MS (DCl/$NH_3$) : m/z : 712/714 (M+$NH_4$)$^+$,

*4-[(2,3,4-tri-O-benzyl)-β-D-benzylglucuronyloxy]-3-chlorobenzyl-4-nitrophenylcarbonat(1*

Der Alkohol 10 (0.40 g, 0.57 mmol) wurde in Ethylacetat (2 ml) und Pyridin (0.2 mol) gelöst. 4-Nitrophenylchloroformiat (0.40 g, 1.97 mmol) wurde hinzugegeben und die Mischung über Nacht gerührt. Die Lösungsmittel wurden unter reduziertem Druck abgedampft. Die Reinigung des Rückstandes durch Säulenchromatographie über Silica Gel 60 H [Hexan : Ethylacetat (7:3 v/v)] ergab 4-[(2,3,4-tri-O-benzyl)-β-D-benzylglucuronyloxy]-3-chloro-benzyl-4-nitrophenylcarbonat(11) (0.23 g, 47 %).
$C_{48}H_{42}ClNO_{12}$, M = 845.5
IR (KBr) ν cm-1 : 3050, 2940, 1735, 1510, 1360, 1320, 1230, 1050, 835, 810.
$^1$H NMR (300 MHz, $CDCl_3$) : 3.40-4.50 (4H, m), 4.55-5.00 (8H, m), 5.12 (1H, d, J = 7 Hz), 5.21 (2H, s), 6.80-8.40 (27H, m).
MS (DCl/$NH_3$) : m/z : 863/865 (M+$NH_4$)$^+$.

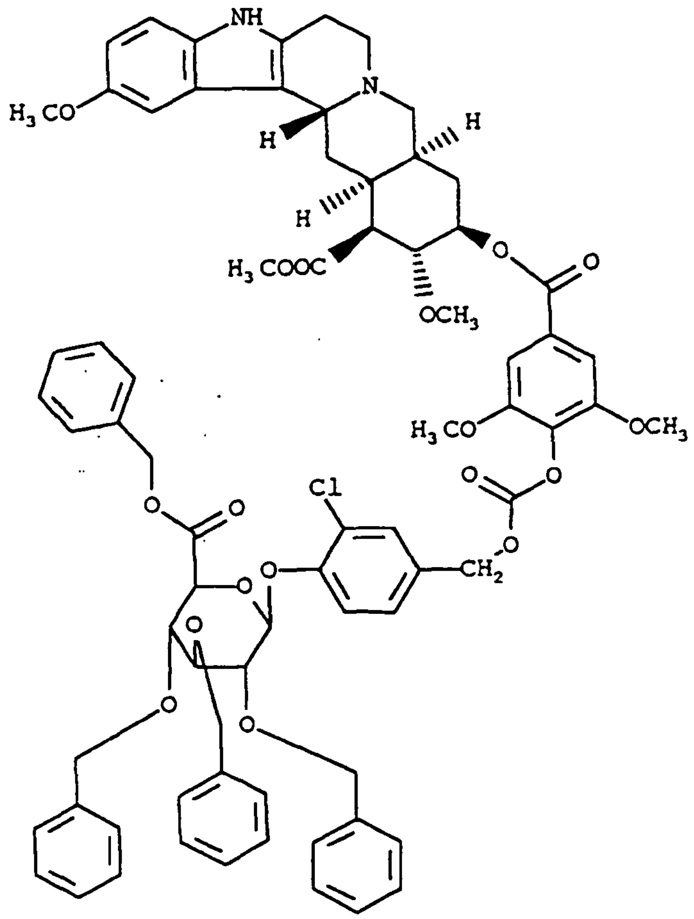

Zu einer gerührten Lösung von L1 (0.20 g, 0.235 mmol) in Dichlormethan (20 ml) und Triethylamin (260 μl) wurde Methyl 18-O-(3,5-dimethoxy-4-hydroxybenzoyl)-reserpat [Lucas et al., J. Am. Chem. Soc., 1959, 81, 1928-1932] (130 mg, 0.22 mmol) hinzugegeben. Die Mischung wurde 22 hrs bei Raumtemperatur gehalten. Die Lösungsmittel wurden unter reduziertem Druck abgedampft. Der Rückstand wurde durch Säulenchromatographie und Silica Gel 60 H [Dichlormethan : Methanol (90:10 v/v)] gereinigt und ergab 12 als farblosen Schaum (0.127 g, 44 %).
$C_{74}H_{75}ClN_2O_{18}$, M = 1314.5
IR (KBr) ν cm-1 : 3050, 2940, 1750, 1720, 1515, 1360, 1320, 1280, 1230, 1050, 835.
$^1H$ NMR (300 MHz, $CDCl_3$) : 1.80-4.50 (19H, m), 3.46 (3H, s), 3.80 (3H, s), 3.82 (3H, s), 3.97 (6H, s), 4.60-5.00 (8H, m), 5.05 (1H, ddd, J = 12.9, 5 Hz), 5.14 (1H, d, J = 7 Hz), 5.22 (2H, s), 6.70-7.90 (28H, m).
MS (FAB, Matrix : Thioglycerol) : 1315/1317 ($m+H^+$).

*Verbindung 25*

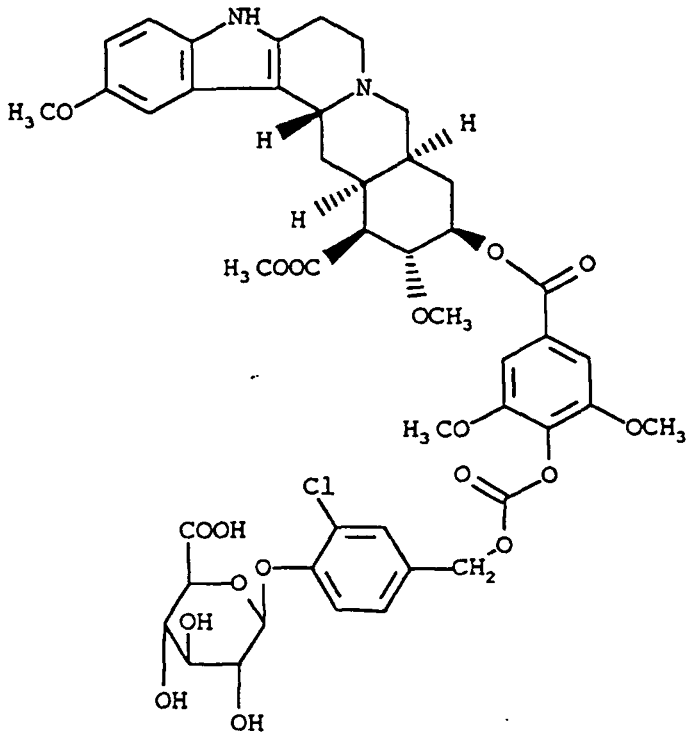

Zu einer Lösung von 12 (0.100 g, 90.07 mmol) wurde Palladium (10 %) auf Aktivkohle (0.2 g) hinzugegeben, und die entstandene Mischung wurde 3 hrs unter Wasserstoff (1 Atm) gehalten. Der Katalysator wurde durch Filtration über Celit entfernt. Das Filtrat wurde unter reduziertem Druck eingedampft. Der Rückstand wurde durch Säulenchromatographie und Silica Gel 60H [Acetonitril : Wasser (92:8 v/v)] gereinigt und ergab Verbindung 25 als farblosen Schaum (0.034 g, 51 %).
$C_{46}H_{51}ClN_2O_{18}$, M = 954,5
IR (KBr) ν cm-1 : 3450, 3050, 2940, 1740, 1725, 1520, 1500, 1360, 1280, 1230, 1050, 825.
$^1H$ NMR (300 MHz, $(CD_3)_2SO$)) : 1.80-4.50 (19H, m), 3.44 (3H, s), 3.79 (3H, s), 3.81 (3H, s), 3.99 (6H, s), 5.04 (1H, ddd, J = 12.9, 5 Hz), 5.12 (1H, d, J = 7 Hz), 5.21 (2H, s), 6.70-7.80 (8H, m).
Ms (FAB, Matrix: Thioglycerol) : 955/957 ($m+H^+$).

Beispielhaft wurde die pharmakologische Wirksamkeit der in den Beispielen 1-25 synthetisierten Glykosyl-Spacer-Wirkstoff Verbindungen (in folgenden Prodrug genannt) in vivo in relevanten tierexperimentellen Systemen getestet. Für die onkologische Indikation wurde ein Modell gewählt, in dem humane Tumore subkutan auf nackte Mäuse transplantiert werden und die erfindungsgemäßen Prodrugs nach Etablierung des Tumors i.v. appliziert wurden.

Beispiel 26:

**Ermittlung der akuten Toxizität:**

Zur Ermittlung der akuten Toxizität wurden Nacktmäusen (CD-1, nu/nu) am Tag 0 unterschiedliche Dosen der Testsubstanz, gelöst in 0.5 ml 5% Glucose-Lösung, über einen Zeitraum von 5 Minuten infundiert. Kontrollgruppen erhalten lediglich 0.5 ml, 5 % Glucose-Lösung. Pro Konzentration der Testsubstanz werden 5 Mäuse verwendet. Am Tag 14 wird die Zahl der überlebenden Mäuse ermittelt und nach der Litchfield Wilcoxon Methode die $LD_{50}$ ermittelt. Die Toxizität der untersuchten Prodrug im Vergleich zum Wirkstoff (Doxorubicin) ist in Tabelle 1 zusammengefaßt.

Tabelle 1: Akute Toxizität in Nacktmäusen

| Substanz | LD50 (mg/kg) |
|---|---|
| 14-O-[4-beta-D-glucuronyloxy)-3-nitro-benzylaminocarbonyl]-doxorubicin | > 1500 |
| Doxorubicin | 20 |

Beispiel 27

**Inhibition des Wachstums von subkutan in der Nacktmaus wachsenden Humantumoren:**

Die wachstumsinhibierende Aktivität der Testsubstanzen wurde in Anlehnung an die von Fiebig et al. (Proc. Europ. Soc. Mod. Oncol. in Cancer Chemother. Pharmacol., Suppl. 9, 18, 1982; Verh. dtsch. Ges. inn. Med. 88, 966, 1982) und Inoue et al. (Cancer Chemother. Pharmacol. 10, 182-186, 1983) beschriebene Methode geprüft. Die getesteten Humantumore werden routinemäßig in Nacktmäusen gehalten und passagiert. Die Tumore werden bei jeder 3. Passage auf ihren humanen Charakter durch Immunhistochemie mit monoklonalen Antikörpern geprüft. Der Tumor wird unter sterilen Bedingungen entfernt und in kleine Stücke von etwa 5-10 $mm^3$ zerschnitten. Je ein Tumorstückchen wird subkutan in die Seite einer Nacktmaus implantiert. Nach ca. 7-14 Tagen ist das Tumorstückchen im umgebenden Gewebe angewachsen und die Tumorgröße A wird mit Hilfe einer Schieblehre und Messung zweier gegenüberliegender Durchmesser (a, b) nach folgender Formel bestimmt:

$$A = a \times b$$

Nach einer erneuten, im Abstand von 3 Tagen vorgenommenen Messung der Tumorgröße werden die Tiere in die Kontrollgruppe und die zu behandelnden Gruppen (jeweils 6 Tiere/Gruppe) randomisiert. Hierbei werden nur Tiere verwendet, bei denen ein progressives Tumorwachstum festgestellt wurde. Beginnend mit dem Tag der Randomisierung (Tag -7) werden die Tiere gemäß dem unten angegebenen Schema mit den Testsubstanzen behandelt. Zweimal pro Woche werden die beiden Tumordurchmesser für jede Maus gemessen und die individuellen Tumorflächen werden entsprechend der oben genannten Formel berechnet.

Nach Beendigung des Experimentes werden die relativen Tümorgrößen für jedes individuelle Tier zum jeweiligen Meßtag gemäß der Formel: $A_r = A_{(Tag\ X\ )}/A_{(Tag\ 0)}$ berechnet. Danach wird der Median der behandelten Gruppe ($A_T$) in Beziehung zum Median der relativen Tumorgröße der Kontrolle ($A_C$) gesetzt und T/C % = $V_T$ / $A_C$ x 100 berechnet. Die statistische Signifikanz des antitumoralen Effektes wird mit Hilfe des Wilcoxon U-Testes bestimmt.

**Ergebnis:**

Tabelle 2: Wirksamkeit gegenüber subkutan in der Nacktmaus wachsenden humanen Colontumoren (LoVo)

| Substanz | Dosis (mg/kg) | Behandlungsschema | T/C (%) | Signifikanz ($p < 0.05$) |
|---|---|---|---|---|
| Prodrug* | 500 | 1xi.v., d0 | 40.0 | + |
| Fusionsprotein<br>+ Prodrug* | 30<br>500 | 1xi.v., d-7<br>1xi.v., d0 | 42.0 | + |
| Doxorubicin | 12 | 1xi.v., d0 | 78.4 | |
| *: 14-O-[4-(beta-D-glucuronyloxy)-3-nitro-benzylaminocarbonyl]-doxorubicin | | | | |

Die in Tabelle 2 präsentierten tierexperimentellen Daten zeigen, daß das Tumorwachstum bei den Tieren, die mit Fusionsprotein und Prodrug oder mit Prodrug allein behandelt wurden, weniger schnell ist (T/C - 40%) als das bei Doxorubicinbehandlung beobachtete Tumorwachstum (T/C - 80%). Die Unterschiede zwischen Doxorubicinbehandlung und Prodrugtherapie bzw. Doxorubicinbehandlung und Fusionsprotein + Prodrugtherapie sind statistisch signifikant ($p < 0.05$).

Überraschenderweise war die Therapie in der Prodrug-Gruppe ebenso effizient wie in der Gruppe, die Fusionsprotein in Kombination mit Prodrug erhielt. Diese unerwartete Beobachtung konnte durch folgende Gewebeanalysen erklärt werden: Histologische Untersuchungen an kryopräservierten LoVo-Tumoren, die zum Zeitpunkt der Prodrug- oder Doxorubicintherapie (Tabelle 2, d0) von tumortragenden Nacktmäusen entnommen wurden, zeigten, daß die Tumore eine ausgedehnte zentrale Nekrose entwickelt hatten. Mittels eines histochemischen Nachweises (Murray et al., the journal of histochemistry and cytochemistry 37, 643-652, 1989) zur Bestimmung funktioneller humaner β-Glucuronidase konnte gezeigt werden, daß innerhalb der Nekrose funktionell aktive humane β-Glucuronidase vorhanden ist; d.h. Zellen, deren Zytoplasmamembran bereits zerstört ist, enthalten noch intrazytoplasmatisch funktionell aktive β-Glucuronidase. Diese in der Nekrose lokalisierte, nicht durch die Zytoplasmamembran geschützte, humane β-Glucuronidase bewirkt die Spaltung der hydrophilen Prodrug zur Drug im LoVo-Tumor.
Dieser unerwartete Befund der endogenen Aktivierung der Prodrug durch die zentrale Nekrose ließ sich bei einer Vielzahl humaner Tumorxenografts (Ovarial-, Brust-, Magen-, Lungen- und Darmkarzinomen) bestätigen. Histologische und histochemische Untersuchungen an Biopsiematerialien aus humanen Karzinomen belegen, daß die zentrale Nekrose kein Artefakt darstellt, der nur bei xenotransplantierten Humantumoren auf Nacktmäusen auftritt, sondern ein weit verbreitetes pathophysiologisches Phänomen darstellt, welches eine breite Einsatzmöglichkeit der Prodrug-Monotherapie beim Menschen erwarten läßt. Tumore, die keinen signifikanten Nekroseanteil entwickeln, können durch die Prodrug-Monotherapie jedoch nicht therapiert werden (Daten nicht gezeigt). Bei solchen Tumoren ist, um die überlegene Wirkung der Prodrugtherapie ausnutzen zu können, die Verwndung eines extrazellulär im Tumor vorlokalisierten Fusionsproteins zwingend nötig (Bsp.: disseminierte Metastasen, kleine nicht nekrotische Primärtumore etc.)

Beispiel 28

Pharmakokinetik von Prodrug und Doxorubicin in tumortragenden Nacktmäusen Zur Evaluierung der Konzentrationen an Prodrug und Doxorubicin in Gewebe und Tumor wurde die Prodrug (500 mg/kg) bzw. Doxorubicin (10 mg/kg) in Nacktmäuse, denen 14 Tage zuvor ein humaner Colontumor (Mz-Sto-1) subkutan implantiert worden war, über einen Zeitraum von 5 min infundiert. Nach der Infusion wurden die Tiere zu unterschiedlichen Zeiten getötet und die Organe bzw. der Tumor entnommen. Zu je 230 mg Gewebe wurde 770 µl 20 mM Phosphat-Puffer, 10 mM Saccharolacton, pH 3.0, hinzugegeben und die Proben mit Hilfe eines Ultraturrax homogenisiert. Zu je 200 µl dieses Homogenates wurden 40 µl 3.3 % Silbemitratlösung sowie 160 µl Acetonitril hinzugegeben. Nach 30minütigem Schütteln der Proben und anschließender Zentrifugation (5 min, 12000 g) wurden 100 µl des Überstandes abgenommen, mit 300 µl Phosphat-Puffer, 10 mM Saccharolacton, pH 6.0, verdünnt, der Gehalt an Prodrug bzw. Doxorubicin mittels automatischer Vorsäulenextraktion über C-18 Bondelut-Kartuschen (AASP) und Hochdruckflüssigkeitschromatographie analysiert.

<u>Ergebnisse:</u>

Tabelle 3: Pharmakokinetik von Prodrug und Doxorubicin in tumortragenden Nacktmäusen

| Substanz | Prodrug (500 mg/kg) | | | Doxorubicin (10 mg/kg) |
|---|---|---|---|---|
| Organ | Zeit nach Applikation (h) | Prodrug (µg/g) | Doxorubicin (µg/g | Doxorubicin (µg/g) |
| **Tumor Mz-Sto-1** | 0.5 | 57,1 | 4.7 | 1.4 |
| | 1.0 | 69,3 | 11.4 | 2.2 |
| | 4.0 | 12,4 | 16.4 | 1.8 |
| | 8.0 | 3,4 | 9.2 | 1.4 |
| **Herz** | 0.5 | 68,9 | 4.8 | 9.1 |
| | 1.0 | 83,3 | 5.1 | 10.7 |
| | 4.0 | 5,8 | 4.2 | 14.3 |
| | 8.0 | 0,7 | 3.2 | 5.8 |
| **Leber** | 0.5 | 131,7 | 11.6 | 23.0 |
| | 1.0 | 165,4 | 13.2 | 23.0 |
| | 4.0 | 27,7 | 8.2 | 17.0 |
| | 8.0 | 6,5 | 6.0 | 9.7 |
| **Lunge** | 0.5 | 156,3 | 6.7 | 12.1 |
| | 1.0 | 186,3 | 10.2 | 15.5 |
| | 4.0 | 7,95 | 9.3 | 21.9 |
| | 8.0 | 0,8 | 5.0 | 15.3 |
| **Niere** | 0.5 | 228,9 | 18.2 | 57.0 |
| | 1.0 | 9373,0 | 53.9 | 39.5 |
| | 4.0 | 162,3 | 14.0 | 30.2 |

(fortgesetzt)

| Substanz | Prodrug (500 mg/kg) | | | Doxorubicin (10 mg/kg) |
|---|---|---|---|---|
| Organ | Zeit nach Applikation (h) | Prodrug (µg/g) | Doxorubicin (µg/g | Doxorubicin (µg/g) |
| | 8.0 | 48,2 | 11.6 | 14.9 |
| **Milz** | 0.5 | 47,0 | 6.8 | 19.4 |
| | 1.0 | 89,1 | 9.7 | 4.5 |
| | 4.0 | 9,9 | 13.5 | 4.8 |
| | 8.0 | 0,3 | 7.3 | 3.9 |
| **Muskel** | 0.5 | 53,0 | 2,0 | 3.5 |
| | 1.0 | 72,9 | 2.9 | 9.6 |
| | 4.0 | 3,8 | 2.7 | 4.7 |
| | 8.0 | 0,6 | 2.0 | 5.2 |

Beispiel 29

Die Wirkung von 14-O-[4-(beta-D-glucuronyloxy)-3-nitro-benzylaminocarbonyl]-doxorubicin (Prodrug) auf die Entzündung wurde in einem Modell zur Hautreaktion vom verzögerten Typ (Delayed Type Hypersensitivity, DTH) entsprechend der von Collins und Mackaness (J. Immunol., 101: 830-845, 1968) beschriebenen Methode untersucht.

In diesem Modell wurden Mäuse mit einer definierten Menge abgetöteter Salmonella typhimurium Bakterien ($10^9$) zweimal im Abstand von einer Woche immunisiert. Drei Wochen nach der Erstimmunisierung wurde eine DTH-Reaktion provoziert, indem ein Salmonella typhimurium Antigen (STA) intraplantar injiziert wurde. Es entwickelte sich ein lokales, entzündliches Infiltrat, das aus Granulozyten, Makrophagen, Lymphozyten sowie eingelagerter interstitieller Flüssigkeit bestand. Das Ausmaß der Reaktion wurde über die Zunahme der Fußschwellung 24 Stunden nach Provokation der Reaktion gemessen.

Die Wirkung der Prodrug auf die Entzündungsreaktion wurde in zwei Behandlungsschemata geprüft. Eine Gruppe erhielt eine einmalige intravenöse Gabe von 500 mg/kg Prodrug, 2 Stunden nach der Injektion von STA, eine weitere Gruppe erhielt drei Injektionen von jeweils 300 mg/kg Prodrug zum Zeitpunkt 2, 5 und 8 Stunden nach der STA Injektion.

Wie Tabelle 4 zeigt, führten beide Behandlungsschemata mit der Prodrug zu einer signifikanten Reduktion der Entzündungsreaktion, wobei die dreimalige Substanzgabe der Standardtherapie mit dem Antiinflammatorikum Ibuprofen leicht überlegen war.

Tabelle 4:

| Gruppe | 24 h Fußschwellung |
|---|---|
| 1. Negativkontrolle | $0.82 \pm 1.32$ |
| 2. Positivkontrolle | $10.7 \pm 3.4$ |
| 3. Prodrug, 500 mg/kg, 1x i.v. (t = + 2 h) | $3.5 \pm 3.9^{x}$ |
| 4. Prodrug, 300 mg/kg, 3x i.v. (t= + 2, + 5, + 8 h) | $1.1 \pm 1.3^{xx}$ |
| 5. Ibuprofen 200 mg/kg, 1 x p.o. (t = 0) | $1.7 \pm 2.2^{xx}$ |

Signifikanzen gegen Positivkontrolle (Gruppe 2) nach Student t-Test
x:p<0.01; xx:p<0.001

Beispiel 30

14-O-[4-(beta-D-Glucuronyloxy)-3-nitro-benzylaminocarbonyl]-doxorubicin (Prodrug) wurde gemäß D. Papahadjopoulos et al. (PNAS, USA 88:11460-11464, 1991) in "stealth"-Liposomen eingeschlossen. Nach i.v.-Injection in CD 1 nu/nu Mäuse war die Plasmahalbwertszeit der in Liposomen eingeschlossenen Prodrug von ≈ 40 Stunden deutlich länger als die Plasmahalbwertszeit der freien Prodrug (≈ 20 min) (Daten nicht gezeigt). Diese signifikante t1/2β Verlängerung führte zu verbesserter pharmakologischen Wirksamkeit. Eine weniger deutliche Plasmahalbwertszeitverlängerung wurde durch Vorinkubation der Prodrug mit 50 g/l humanem Serumalbumin oder humanem sauren alpha-1 Glykoprotein erzielt.

## Patentansprüche

**1.** Verbindung der Formel I

$$\text{Glykosyl-Y[-C(= Y)-X-]}_p\text{W(R)}_n\text{-X-C(=Y)-Wirkstoff} \quad \text{(I)}$$

worin

Glykosyl ein enzymatisch abspaltbarer alpha- oder beta-O-glycosidisch verknüpfter D-Glucuronyl-, D-Glucopyranosyl-, D-Galactopyranosyl, N-Acetyl-D-glucosaminyl-, N-Acetyl-D-galactosaminyl-, D-Mannopyranosyl- oder L-Fucopyranosylrest ist,
W ein Phenylrest oder ein mehrfach substituierter Phenylrest, bei dem die Substituenten
R unabhängig oder gleich H, Methyl, Methoxy, Cärboxy, Methyloxycarbonyl, CN, Hydroxy, Nitro, Fluor, Chlor, Brom, Sulfonyl, Sulfonamid oder Sulfon ($C_{1-4}$)-alkylamid sind,
P 0 oder 1,
n 1 - 4,
X O, NH, methylenamino oder methylen-($C_{1-4}$)-alkylamino und
Y O oder NH ist, und

Wirkstoff eine über eine Hydroxy-, Amino- oder Iminogruppe verknüpfte Verbindung ist, **dadurch gekennzeichnet, daß** Wirkstoff ein bei p = 0 nicht über 3'-Aminogruppen verknüpftes Anthrazyklin, vorzugsweise Doxorubicin, 4'-epi-Doxorubicin, 4- oder 4'-Desoxydoxorubicin,
oder eine Verbindung vorzugsweise aus der Gruppe
Etoposide, N-Bis-(2-chlorethyl)-4-hydroxyanilin, 4-Hydroxycyclophosphamid, Vindesin, Vinblastin, Vincristin, 5-Fluorouracil, 5-Fluorouridin, 5-Fluorocytidin, Methotrexat, Mitomycin C, Mitoxantron, Camptothecin, mAMSA, Taxol, Nocodaxol, Colchicin, Cyclophosphamid, Rachelmycin, Cisplatin, Melphalan, Bleomycin, Stickstoff-Senfgas, Phosphoramid-Senfgas, Verrucarin A, Neocarzinostatin, Calicheamicin, Dynemicin, Esperamicin A, Quercetin, Genistein, Erbstatin, Tyrphostin, Rohitukinderivat,
oder deren durch eine oder mehrere Hydroxy-, Amino- oder Iminogruppen zusätzlich substituierte Derivate bedeutet, wobei Wirkstoffe bevorzugt sind, die schon eine Hydroxy-, Amino- oder Iminogruppe tragen.

**2.** Verbindung nach Anspruch 1, **dadurch gekennzeichnet, dass**

W ein Phenylrest oder ein monosubstituierter Phenylrest, bei dem einer der Substituenten
R Methoxy, Methyloxycarbonyl, CN, Hydroxy, Nitro, Fluor, Chlor, Brom, Sulfon oder Sulfanoyl ist, die übrigen Wasserstoff sind, und
p, X, Y und Wirkstoff wie in Anspruch 1 definiert sind.

**3.** Verbindung nach Anspruch 1, **dadurch gekennzeichnet, daß** der Wirkstoff Doxorubicin, 4'-epi-Doxorubicin, 4- oder 4'-Desoxydoxorubicin ist.

**4.** Verbindung nach Anspruch 1, **dadurch gekennzeichnet, daß** die Verbindung
4'-O-[4-(alpha-D-Glucopyranosyloxy)-phenylaminocarbonyl]- etoposid,
4'-O-[4-(beta-D-Glucopyranosyloxy)-phenylaminocarbonyl]- etoposid,
4'-O-[4-(alpha-D-Galactopyranosyloxy)-phenylaminocarbonyl] -etoposid,
4'-O-[4-(beta-D-Glucuronyloxy)-phenylaminocarbonyl]-etoposid,
4'-O-[4-(beta-D-Glucuronyloxy)-3-nitro-benzylaminocarbonyl]-etoposid,
4'-O-[4-(beta-D-Glucuronyloxy)-3-chlorbenzylaminocarbonyl]-etoposid,
1-N-[4-(beta-D-Glucuronyloxy)-benzyloxycarbonyl]-mitomycin C,
14-O-[4-(beta-D-Glucuronyloxy)-3-nitro-benzylaminocarbonyl]-doxorubicin,
4-O-[4-(beta-D-Glucuronyloxy)-benzylaminocarbonyl]4- hydroxy-1-N-(bis-2-chlorethyl)-anilin, oder
N-[N-(alpha-D-Galactopyranosyloxycarbonyl)-4-hydroxymethyloxycarbonylanilin]-doxorubicin ist.

5. Verbindung nach Anspruch 1, **dadurch gekennzeichnet, daß** die Verbindung 14-O-[4-(beta-D-Glucuronyloxy)-3-nitro-benzylaminocarbonyl]-doxorubicin ist,

## Claims

1. A compound of the formula I

glycosyl-Y[-C(=Y)-X-]$_p$-W(R)$_n$-X-C(=Y)-active substance (I)

in which

glycosyl is an alpha- or beta-O-glycosidically linked D-glucuronyl, D-glucopyranosyl, D-galactopyranosyl, N-acetyl- D-glucosaminyl, N-acetyl-D-galactosaminyl, D-mannopyranosyl or L-fucopyranosyl radical which can be cleaved off enzymatically,
W is a phenyl radical or a polysubstituted phenyl radical in which the substituents
R are, independently or identically, H, methyl, methoxy, carboxyl, methyloxycarbonyl, CN, hydroxyl, nitro, fluorine, chlorine, bromine, sulfo, sulfamoyl or ($C_{1-4}$)- alkylsulfamoyl,
p is 0 or 1,
n is 1-4,
X is O, NH, methyleneamino or methylene($C_{1-4}$)-alkylamino and
Y is O or NH, and

active substance is a compound which is linked via a hydroxyl, amino or imino group, wherein active substance is an anthracycline which is not linked by 3'-amino groups when p = 0, preferably doxorubicin, 4'-epidoxorubicin, 4- or 4'-deoxydoxorubicin, or a compound preferably selected from the group comprising etoposides, N,N-bis(2-chloroethyl)-4-hydroxyaniline, 4-hydroxycyclophosphamide, vindesine, vinblastine, vincristine, 5-fluorouracil, 5-fluorouridine, 5-fluorocytidine, methotrexate, mitomycin C, mitoxantrone, camptothecin, m-AMSA, taxol, nocodazole, colchicine, cyclophosphamide, rachelmycin, cisplatin, melphalan, bleomycin, nitrogen mustard, phosphoramide mustard, verrucarin A, neocarcinostatin, calicheamicin, dynemicin, esperamicin A, quercetin, genistein, erbstatin, tyrphostin, rohitukin derivative,
or their derivatives additionally substituted by one or more hydroxyl, amino or imino groups, where preferred active substances already have a hydroxyl, amino or imino group.

2. A compound as claimed in claim 1, wherein

W is a phenyl radical or a monosubstituted phenyl radical in which one of the substituents
R is methoxy, methyloxycarbonyl, CN, hydroxyl, nitro, fluorine, chlorine, bromine, sulfo or sulfamoyl, and the others are hydrogen, and
p, X, Y and active substance are as defined in claim 1.

3. A compound as claimed in claim 1, wherein the active substance is doxorubicin, 4'-epidoxorubicin, 4- or 4'-deoxydoxorubicin.

4. A compound as claimed in claim 1, wherein the compound is 4'-O-[4-(alpha-D-glucopyranosyloxy)phenylaminocarbonyl]etoposide, 4'-O-[4-(beta-D-glucopyranosyloxy)phenylaminocarbonyl]etoposide, 4'-O-[4-(alpha-D-galactopyranosyloxy)phenylaminocarbonyl]etoposide, 4'-O-[4-(beta-D-glucuronyloxy)phenylaminocarbonyl]etoposide, 4'-O-[4-(beta-D-glucuronyloxy)-3-nitrobenzylaminocarbonyl]etoposide, 4'-O-[4-(beta-D-glucuronyloxy)-3-chlorobenzylaminocarbonyl]etoposide, 1-N-[4-(beta-D-glucuronyloxy)benzyloxycarbonyl]mitomycin C, 14-O-[4-(beta-D-glucuronyloxy)-3-nitrobenzylaminocarbonyl]-doxorubicin, 4-O-[4-(beta-D-glucuronyloxy)benzylaminocarbonyl]-4-hydroxy-1-N-bis(2-chloroethyl)aniline, N-[N-(alpha-D-galactopyranosyloxy-carbonyl)-4-hydroxymethyloxy-carbonylaniline]doxorubicin.

5. A compound as claimed in claim 1, wherein the compound is 14-O-[4-(beta-D-glucuronyloxy)-3-nitro-4-hydroxymethylaminocarbonylaniline]doxorubicin.

## Revendications

1. Composé de formule I

$$Glycosyl\text{-}Y[\text{-}C(=Y)\text{-}X]_pW(R)_nW(R)_n\text{-}X\text{-}C(=Y)\text{-}principe\ actif \quad (I)$$

dans laquelle
"glycosyl" représente un résidu D-glucuronyle, D-glucopyrannosyle, D-galactopyrannosyle, N-acétyl-D-glucosaminyle, N-acétyl-D-galactosaminyle, D-mannopyrannosyle ou L-fucopyrannosyle à liaison alpha- ou bêta-o-glycosidique, pouvant subir une dissociation enzymatique,
W est un radical phényle ou un radical phényle plusieurs fois substitué, dans lequel les substituants R représentent chacun, indépendamment des autres, H, un groupe méthyle, méthoxy, carboxy, méthyloxycarbonyle, CN, hydroxy, nitro, fluoro, chloro, bromo, sulfo, sulfamayle ou sulfamoyle (alkyle en $C_1$-$C_4$),
p vaut 0 ou 1,
n vaut 1-4,
X est O, NH, le groupe méthylène-amino ou un groupe méthylène-(alkyle en $C_1$-$C_4$)amino, et
Y est 0 ou NH,
et le principe actif est un composé lié par l'intermédiaire d'un groupe hydroxy, amino ou imino,
**caractérisé en ce que** le principe actif est une anthracycline qui, pour p = 0, n'est pas liée par l'intermédiaires de groupes 3'-amino, de préférence la doxorubicine, la 4'-épi-doxorubicine, la 4- ou la 4'-désoxydoxorubicine, ou un composé de préférence du groupe consistant en l'étoposide, la N,N-bis-(2-chloroéthyl)-4-hydroxyaniline, le 4-hydroxycyclophosphamide, la vindésine, la vinblastine, la vincristine, le 5-fluorouracile, la 5-fluorouridine, la 5-fluorocytidine, le méthotréxate, la mitomycine C, le mitoxantron, la camptothécine, le mAMSA, le taxol, le nocodazol, la colchicine, le cyclophosphamide, la rachelmycine, le cisplatine, le melphalan, la bléomycine, le gaz moutarde à l'azote, le gaz moutarde au phosphoramide, la verrucarine A, la néocarzinostatine, la calichéamicine, la dynémicine, l'espéramicine A, la quercétine, la génistéine, l'erbstatine, la tyrphostine, le dérivé de rohitukine, ou leurs dérivés substitués en outre par un ou plusieurs groupes hydroxy, amino ou imino, auquel cas on préfère les principes actifs qui portent déjà un groupe hydroxy, amino ou imino.

2. Composé selon la revendication 1, **caractérisé en ce que**
W est un groupe phényle ou un groupe phényle monosubstitué, dans lequel l'un des substituants
R est un groupe méthoxy, méthyloxycarbonyle, CN, hydroxy, nitro, fluoro, chloro, bromo, sulfo ou sulfamoyle, les autres étant des atomes d'hydrogène, et
p, X, Y et principe actif ont les définitions données dans la revendication 1.

3. Composé selon la revendication 1, **caractérisé en ce que** le principe actif est la doxorubicine, la 4'-épi-doxorubicine, la 4- ou la 4'-désoxydoxorubicine.

4. Composé selon la revendication 1, **caractérisé en ce que** le composé est
le 4'-O-[4-(alpha-D-glucopyrannosyloxy)-phénylaminocarbonyl]-étoposide,
le 4'-O-[4-(bêta-D-glucopyrannosyloxy)-phénylaminocarbonyl]-étoposide,
le 4'-O-[4-(alpha-D-galactopyrannosyloxy)-phénylaminocarbonyl]-étoposide,
le 4'-O-[4-(bêta-D-galactopyrannosyloxy)-phénylaminocarbonyl]-étoposide,
le 4'-O-[4-(bêta-D-glucuronyloxy)-3-nitro-benzylaminocarbonyl]-étoposide,
le 4'-O-[4-(bêta-D-glucuronyloxy)-3-chloro-benzylaminocarbonyl]-étoposide,
la 1-N-[4-(bêta-D-glucuronyloxy)-benzyloxycarbonyl]-mitomycine C,
la 14-O-[4-(bêta-D-glucuronyloxy)-3-nitro-benzylaminocarbonyl]-doxorubicine,
la 4-O-[4-(bêta-D-glucuronyloxy)benzylaminocarbonyl]-4-hydroxy-1-N-(bis-2-chloroéthyl)-aniline, ou
la N-[N-(alpha-D-galactopyrannosyloxycarbonyl)-4-hydroxyméthyloxycarbonylaniline]-doxorubicine.

5. Composé selon la revendication 1, **caractérisé en ce que** le composé est la 14-O-[4-(bêta-D-glucuronyloxy)-3-nitro-benzylaminocarbonyl]-doxorubicine.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 8101145 A
- US 4481195 A
- WO 8807378 A
- US P4975278 A
- EP 0441218 A2
- EP 89119710 A
- WO 9117748 A


### In der Beschreibung aufgeführte Nicht-Patentliteratur


- **Sweeney ; Mitarbeiter.** *Cancer Research,* 1971, vol. 31, 477-478
- **Young et al.** *Cancer,* 1976, vol. 38, 1887-1895
- **Baba et al.** *Gann,* 1978, vol. 69, 283-284
- *Journal of Medicinal Chemistry,* 1981, vol. 24, 479-480
- **Baba, T. et al.** *Gann,* 1978, vol. 69, 283-284
- **Philpott et al.** *Surgery,* 1973, vol. 74, 51
- *Cancer Res.,* 1974, vol. 34, 2159
- **von Bosslet et al.** *Br. J. Cancer,* 1992, vol. 65, 234-238
- **Florent et al.** *Int. Carbohydr. Symposium,* 1992, 297
- **Gesson et al.** *Int. Carbohydr. Symposium,* 1992, 298
- **Andrianomenjanahary et al.** *Int. Carbohydr. Symposium,* 1992, 299
- **Gerdes et al.** *Amer. J. Pathol.,* 1991, vol. 138, 867-873
- **Bottomley et al.** *Brit. J. Pharmacol.,* 1988, vol. 93, 627-635
- **Ratte Schorlemmer et al.** *Exptl. Clin. Res.,* 1991, vol. XVII (10/11), 471-483
- **Maus Dickneite et al.** *Infect. Immun.,* 1984, vol. 44 (1), 168-174
- **Maus Okayasu et al.** *Gastroenterology,* 1990, vol. 98, 694-702
- **Schorlemmer et al.** *Drugs Exptl. Clin. Res.,* 1991, vol. XVII (10/11), 461-469
- **Schorlemmer et al.** *Int. J. Immunother,* 1991, vol. VII (4), 169-180
- **Lucas et al.** *J. Am. Chem. Soc.,* 1959, vol. 81, 1928-1932
- **Fiebig et al.** *Proc. Europ. Soc. Mod. Oncol. in Cancer Chemother. Pharmacol., Suppl,* 1982, vol. 9, 18
- *Verh. dtsch. Ges. inn. Med.,* 1982, vol. 88, 966
- **Inoue et al.** *Cancer Chemother. Pharmacol.,* 1983, vol. 10, 182-186
- **Murray et al.** *the journal of histochemistry and cytochemistry,* 1989, vol. 37, 643-652
- **Collins ; Mackaness.** *J. Immunol.,* 1968, vol. 101, 830-845
- **D. Papahadjopoulos et al.** *PNAS, USA,* 1991, vol. 88, 11460-11464